# EUROPEAN PATENT SPECIFICATION

(11) **EP 3 668 970 B1**
(45) Date of publication and mention of the grant of the patent: **02.12.2026**
(21) Application number: 18779168.6
(22) Date of filing: 11.09.2018
(51) Int. Cl.: C12N 9/10, C12P 19/04, C12P 19/18

(54) **ENGINEERED GLUCOSYLTRANSFERASES**
MANIPULIERTE GLUCOSYLTRANSFERASEN
GLUCOSYLTRANSFÉRASES MODIFIÉES

(30) Priority: 13.09.2017 US 201762557840 P
(43) Date of publication of application: 24.06.2020
(73) Proprietor: Nutrition & Biosciences USA 4, Inc., Rochester NY 14623 (US)
(72) Inventor: LI, Yougen, Glen Mills, Pennsylvania 19342 (US); SEMKE, Ellen D., Newark, Delaware 19713 (US); CHENG, Qiong, Wilmington, Delaware 19803 (US); LAI, Jian Ping, Wallingford, Pennsylvania 19086 (US)
(74) Representative: Dehns
(86) International application number: PCT/US2018/050354
(87) International publication number: WO 2019/055377

(56) References cited:
- WO-A1-2018/052942
- WO-A2-2019/094357
- US-A1- 2014 087 431
- DATABASE UniProt [online] 12 April 2017 (2017-04-12), "SubName: Full=Dextransucrase {ECO:0000313|EMBL:SDB26833.1};", XP002786137, retrieved from EBI accession no. UNIPROT:A0A1G6C1V1 Database accession no. A0A1G6C1V1
- DATABASE Geneseq [online] 23 February 2017 (2017-02-23), "S. salivarius GTF polypeptide deletion fragment SEQ: 97.", XP002786138, retrieved from EBI accession no. GSP:BDL33365 Database accession no. BDL33365
- DATABASE UniProt [online] 8 June 2016 (2016-06-08), "SubName: Full=KxYKxGKxW signal domain protein {ECO:0000313|EMBL:KXI10206.1};", XP002786139, retrieved from EBI accession no. UNIPROT:A0A135YLE3 Database accession no. A0A135YLE3

## Description

This application claims the benefit of U.S. Provisional Application No. 62/557,840 (filed September 13, 2017).

### FIELD

The present disclosure is in the field of enzyme catalysis. For example, the disclosure pertains to glucosyltransferase enzymes with modified amino acid sequences. Such modified enzymes have improved product yield properties, for example.

### REFERENCE TO SEQUENCE LISTING SUBMITTED ELECTRONICALLY

The official copy of the sequence listing is submitted electronically via EFS-Web as an ASCII formatted sequence listing with a file named 20180911_CL6613WOPCT_SequenceListing_ST25 created on September 11, 2018, and having a size of about 406 kilobytes and is filed concurrently with the specification. The sequence listing contained in this ASCII-formatted document is part of the specification.

### BACKGROUND

Driven by a desire to use polysaccharides in various applications, researchers have explored for polysaccharides that are biodegradable and that can be made economically from renewably sourced feedstocks. One such polysaccharide is alpha-1,3-glucan, an insoluble glucan polymer characterized by having alpha-1,3-glycosidic linkages. This polymer has been prepared, for example, using a glucosyltransferase enzyme isolated from *Streptococcus salivarius* (Simpson et al., Microbiology 141:1451-1460, 1995). Also for example, U.S. Patent No. 7000000 disclosed the preparation of a spun fiber from enzymatically produced alpha-1,3-glucan. Various other glucan materials have also been studied for developing new or enhanced applications. For example, U.S. Patent Appl. Publ. No. 2015/0232819 discloses enzymatic synthesis of several insoluble glucans having mixed alpha-1,3 and -1,6 linkages.

While these and other advances have been made in producing glucan polymers using glucosyltransferase enzymes, less attention appears to have been drawn to improving the glucan yields of such enzymes. Addressing this technological gap, disclosed herein are glucosyltransferases engineered to have modified amino acid sequences endowing these enzymes with enhanced glucan production properties.

DATABASE UniProt 12 April 2017 (2017-04-12), "SubName: Full=Dextransucrase {ECO:0000313|EMBL:SDB26833.1};" discloses a glycosyltransferase from *Streptococcus Henryi* which has 57.3% identity with SEQ ID NO; 62 and comprises the mutations 588R, 741Y, and 1188T.

DATABASE Geneseq 23 February 2017 (2017-02-23), "S. salivarius GTF polypeptide deletion fragment SEQ: 97." discloses a glycosyltransferase from *Streptococcus salivarius* which has 93.8 % identity with SEQ ID NO: 62 and has a deletion at position 588, and 631T, 710S, 741E, and 1188T.

DATABASE UniProt 8 June 2016 (2016-06-08), "SubName: Full=KxYKxGKxW signal domain protein {ECO:0000313|EMBL:KXI10206.1};" discloses a glycosyltransferase from *Streptococcus pasteuranius* which has 54% identity with SEQ ID NO: 62 and has deletions at positions 588 and 741.

US 2014/087431 A1 discloses reaction solutions comprising water, sucrose and a glycosyltransferase enzyme that synthesises poly alpha-1,3-glucan having at least 50% alpha-1,3-glycosidic linkages and a number average degree of polymerisation of at least 100.

WO 2018/052942 A1 discloses glucosyltransferases with modified amino acid sequences which exhibit improved alpha-glucan product yields and/or lower sucrose yields.

WO 2019/094357 A2 discloses compositions comprising aggregates of insoluble alpha glucan with alpha-1,3-glycosidic linkages. Non-native glucosyltransferases comprising amino acid substitutions are disclosed.

### SUMMARY

In one embodiment, the present disclosure concerns a non-native glucosyltransferase comprising at least two amino acid substitutions at positions corresponding with amino acid residues Gln-588, Phe-607, or Arg-741 of SEQ ID NO:62, wherein the non-native glucosyltransferase synthesizes alpha-glucan comprising 1,3-linkages, and wherein the non-native glucosyltransferase has: (i) an alpha-glucan yield that is higher than the alpha-glucan yield of a second glucosyltransferase that only differs from the non-native glucosyltransferase at the substitution positions, and/or (ii) a leucrose yield that is lower than the leucrose yield of the second glucosyltransferase;
wherein the glucosyltransferase comprises a catalytic domain that is at least 90% identical to residues 55-960 of SEQ ID NO:4; and
wherein:
   (a) the amino acid substitution at the position corresponding with amino acid residue Gln-588 is with a Leu, Ala, or Val residue;
   (b) the amino acid substitution at the position corresponding with amino acid residue Phe-607 is with a Trp or Asn residue; and/or
   (c) the amino acid substitution at the position corresponding with amino acid residue Arg-741 is with a Ser or Thr residue.

In another embodiment, the present disclosure concerns a polynucleotide comprising a nucleotide sequence encoding a non-native glucosyltransferase as presently disclosed, wherein one or more regulatory sequences are operably linked to the nucleotide sequence, and preferably wherein the one or more regulatory sequences include a promoter sequence.

In another embodiment, the present disclosure concerns a reaction composition comprising water, sucrose, and a non-native glucosyltransferase as presently disclosed.

In another embodiment, the present disclosure concerns a method of producing alpha-glucan comprising: (a) contacting at least water, sucrose, and a non-native glucosyltransferase as presently disclosed, whereby alpha-glucan is produced; and b) optionally, isolating the alpha-glucan produced in step (a).

### BRIEF DESCRIPTION OF THE SEQUENCES

**Table 1. Summary of Nucleic Acid and Protein SEQ ID Numbers^{b}**

| Description | Nucleic acid SEQ ID NO. | Protein SEQ ID NO. |
|---|---|---|
| GTF 0874, *Streptococcus sobrinus.* The first 156 amino acids of the protein are deleted compared to GENBANK Identification No. 450874; a start methionine is included. | 1 ^{a} | 2 (1435 aa) |
| GTF 6855, *Streptococcus salivarius* SK126. The first 178 amino acids of the protein are deleted compared to GENBANK Identification No. 228476855 (Acc. No. ZP_04061500.1); a start methionine is included. | 3^{a} | 4 (1341 aa) |
| GTF 2379, *Streptococcus salivarius.* The first 203 amino acids of the protein are deleted compared to GENBANK Identification No. 662379; a start methionine is included. | 5^{a} | 6 (1247 aa) |
| GTF 7527 or GTFJ, *Streptococcus salivarius.* The first 42 amino acids of the protein are deleted compared to | 7^{a} | 8 (1477 aa) |
| GENBANK Identification No. 47527; a start methionine is included. | | |
| GTF 1724, *Streptococcus downei.* The first 162 amino acids of the protein are deleted compared to GENBANK Identification No. 121724; a start methionine is included. | 9 ^{a} | 10 (1436 aa) |
| GTF 0544, *Streptococcus mutans.* The first 164 amino acids of the protein are deleted compared to GENBANK Identification No. 290580544; a start methionine is included. | 11 ^{a} | 12 (1313 aa) |
| GTF 5926, *Streptococcus dentirousetti.* The first 144 amino acids of the protein are deleted compared to GENBANK Identification No. 167735926; a start methionine is included. | 13^{a} | 14 (1323 aa) |
| GTF 4297, *Streptococcus oralis.* The first 228 amino acids of the protein are deleted compared to GENBANK Identification No. 7684297; a start methionine is included. | 15^{a} | 16 (1348 aa) |
| GTF 5618, *Streptococcus sanguinis.* The first 223 amino acids of the protein are deleted compared to GENBANK Identification No. 328945618; a start methionine is included. | 17^{a} | 18 (1348 aa) |
| GTF 2765, unknown *Streptococcus* sp. C150. The first 193 amino acids of the protein are deleted compared to GENBANK Identification No. 322372765; a start methionine is included. | 19^{a} | 20 (1340 aa) |
| GTF 0427, *Streptococcus sobrinus.* The first 156 amino acids of the protein are deleted compared to GENBANK Identification No. 940427; a start methionine is included. | 25^{a} | 26 (1435 aa) |
| GTF 2919, *Streptococcus salivarius* PS4. The first 92 amino acids of the protein are deleted compared to GENBANK Identification No. 383282919; a start methionine is included. | 27^{a} | 28 (1340 aa) |
| GTF 2678, *Streptococcus salivarius* K12. The first 188 amino acids of the protein are deleted compared to GENBANK Identification No. 400182678; a start methionine is included. | 29^{a} | 30 (1341 aa) |
| GTF 3929, *Streptococcus salivarius* JIM8777. The first 178 amino acids of the protein are deleted compared to GENBANK Identification No. 387783929; a start methionine is included. | 33^{a} | 34 (1341 aa) |
| GTF 3298, *Streptococcus* sp. C150. The first 209 amino acids of the protein are deleted compared to GENBANK Identification No. 322373298; a start methionine is included. | | 59 (1242 aa) |
| Wild type GTFJ, *Streptococcus salivarius.* GENBANK Identification No. 47527. | | 60 (1518 aa) |
| Wild type GTF corresponding to GTF 2678, *Streptococcus salivarius* K12. | | 61 (1528 aa) |
| Wild type GTF corresponding to GTF 6855, *Streptococcus salivarius* SK126. | | 62 (1518 aa) |
| Wild type GTF corresponding to GTF 2919, *Streptococcus salivarius* PS4. | | 63 (1431 aa) |
| Wild type GTF corresponding to GTF 2765, unknown *Streptococcus* sp. C150. | | 64 (1532 aa) |
| Shorter version of GTF 7527, *Streptococcus salivarius,* (also referred to as "7527-NT" herein. The first 178 amino acids of the protein are deleted compared to GENBANK Identification No. 47527; a start methionine is included. | | 65 (1341 aa) |
| Terminator sequence added to pHY300PLK to derive the pHYT vector. | 67 | |
| Aclqlu1 alpha-glucosidase. | | 68 (971 aa) |
| Nfiglu1 alpha-glucosidase. | | 69 (969 aa) |
| Ncrglu1 alpha-glucosidase. | | 70 (1022 aa) |
| TauSec098_b alpha-glucosidase. | | 71 (1012 aa) |
| TauSec098_c alpha-glucosidase. | | 72 (984 aa) |
| TauSec098_d alpha-glucosidase. | | 73 (984 aa) |
| TauSec099 alpha-glucosidase. | | 74 (973 aa) |
| BloGlu1 alpha-glucosidase. | | 75 (604 aa) |
| BloGlu2 alpha-glucosidase. | | 76 (604 aa) |
| BloGlu3 alpha-glucosidase. | | 77 (604 aa) |
| BpsGlu1 alpha-glucosidase. | | 78 (585 aa) |
| BthGlu1 alpha-glucosidase. | | 79 (601 aa) |
| BbrGlu2 alpha-glucosidase. | | 80 (662 aa) |
| BbrGlu5 alpha-glucosidase. | | 81 (606 aa) |

| | | |
|---|---|---|
| ^{a} This DNA coding sequence is codon-optimized for expression in *E. coli* and is merely disclosed as an example of a suitable coding sequence. ^{b} SEQ ID NOs:21-24, 31, 32, 35-58 and 66 are intentionally not included in this table and merely serve as placeholders. | | |

### DETAILED DESCRIPTION

Unless otherwise disclosed, the terms "a" and "an" as used herein are intended to encompass one or more (i.e., at least one) of a referenced feature.

Where present, all ranges are inclusive and combinable, except as otherwise noted. For example, when a range of "1 to 5" is recited, the recited range should be construed as including ranges "1 to 4", "1 to 3", "1-2", "1-2 & 4-5", "1-3 & 5", and the like.

The terms "alpha-glucan", "alpha-glucan polymer" and the like are used interchangeably herein. An alpha-glucan is a polymer comprising glucose monomeric units linked together by alpha-glycosidic linkages. In typical embodiments, an alpha-glucan herein comprises at least about 80%, 81%, 82%, 83%, 84%, 85%, 86%, 87%, 88%, 89%, 90%, 91%, 92%, 93%, 94%, 95%, 96%, 97%, 98%, 99%, or 100% alpha-glycosidic linkages. Examples of alpha-glucan polymers herein include alpha-1,3-glucan.

The terms "poly alpha-1,3-glucan", "alpha-1,3-glucan", "alpha-1,3-glucan polymer" and the like are used interchangeably herein. Alpha-1,3-glucan is a polymer comprising glucose monomeric units linked together by glycosidic linkages, typically wherein at least about 50% of the glycosidic linkages are alpha-1,3. Alpha-1,3-glucan in certain embodiments comprises at least 90% or 95% alpha-1,3 glycosidic linkages. Most or all of the other linkages in alpha-1,3-glucan herein typically are alpha-1,6, though some linkages may also be alpha-1,2 and/or alpha-1,4.

The terms "glycosidic linkage", "glycosidic bond", "linkage" and the like are used interchangeably herein and refer to the covalent bond that joins a carbohydrate (sugar) molecule to another group such as another carbohydrate. The term "alpha-1,3-glycosidic linkage" as used herein refers to the type of covalent bond that joins alpha-D-glucose molecules to each other through carbons 1 and 3 on adjacent alpha-D-glucose rings. The term "alpha-1,6-glycosidic linkage" as used herein refers to the covalent bond that joins alpha-D-glucose molecules to each other through carbons 1 and 6 on adjacent alpha-D-glucose rings. The glycosidic linkages of a glucan polymer herein can also be referred to as "glucosidic linkages". Herein, "alpha-D-glucose" will be referred to as "glucose".

The glycosidic linkage profile of an alpha-glucan herein can be determined using any method known in the art. For example, a linkage profile can be determined using methods using nuclear magnetic resonance (NMR) spectroscopy (e.g., ¹³C NMR or¹H NMR). These and other methods that can be used are disclosed in, for example, Food Carbohydrates: Chemistry, Physical Properties, and Applications (S. W. Cui, Ed., Chapter 3, S. W. Cui, Structural Analysis of Polysaccharides, Taylor & Francis Group LLC, Boca Raton, FL, 2005).

The "molecular weight" of large alpha-glucan polymers herein can be represented as weight-average molecular weight (Mw) or number-average molecular weight (Mn), the units of which are in Daltons or grams/mole. Alternatively, the molecular weight of large alpha-glucan polymers can be represented as DP_{w} (weight average degree of polymerization) or DPₙ (number average degree of polymerization). The molecular weight of smaller alpha-glucan polymers such as oligosaccharides typically can be provided as "DP" (degree of polymerization), which simply refers to the number of glucoses comprised within the alpha-glucan. Various means are known in the art for calculating these various molecular weight measurements such as with high-pressure liquid chromatography (HPLC), size exclusion chromatography (SEC), or gel permeation chromatography (GPC).

The term "sucrose" herein refers to a non-reducing disaccharide composed of an alpha-D-glucose molecule and a beta-D-fructose molecule linked by an alpha-1,2-glycosidic bond. Sucrose is known commonly as table sugar.

The terms "leucrose" and "D-glucopyranosyl-alpha(1-5)-D-fructopyranose" are used interchangeably herein and refer to a disaccharide containing an alpha-1,5 glucosyl-fructose linkage.

The terms "glucosyltransferase", "glucosyltransferase enzyme", "GTF", "glucansucrase" and the like are used interchangeably herein. The activity of a glucosyltransferase herein catalyzes the reaction of the substrate sucrose to make the products alpha-glucan and fructose. Other products (by-products) of a GTF reaction can include glucose, various soluble gluco-oligosaccharides, and leucrose. Wild type forms of glucosyltransferase enzymes generally contain (in the N-terminal to C-terminal direction) a signal peptide (which is typically removed by cleavage processes), a variable domain, a catalytic domain, and a glucan-binding domain. A glucosyltransferase herein is classified under the glycoside hydrolase family 70 (GH70) according to the CAZy (Carbohydrate-Active EnZymes) database (Cantarel et al., Nucleic Acids Res. 37:D233-238, 2009).

The term "glucosyltransferase catalytic domain" herein refers to the domain of a glucosyltransferase enzyme that provides alpha-glucan-synthesizing activity to a glucosyltransferase enzyme. A glucosyltransferase catalytic domain typically does not require the presence of any other domains to have this activity.

The terms "enzymatic reaction", "glucosyltransferase reaction", "glucan synthesis reaction", "reaction composition", "reaction formulation" and the like are used interchangeably herein and generally refer to a reaction that initially comprises water, sucrose, at least one active glucosyltransferase enzyme, and optionally other components. Components that can be further present in a glucosyltransferase reaction typically after it has commenced include fructose, glucose, leucrose, soluble gluco-oligosaccharides (e.g., DP2-DP7) (such may be considered as products or by-products, depending on the glucosyltransferase used), and/or insoluble alpha-glucan product(s) of DP8 or higher (e.g., DP100 and higher). It would be understood that certain glucan products, such as alpha-1,3-glucan with a degree of polymerization (DP) of at least 8 or 9, are water-insoluble and thus not dissolved in a glucan synthesis reaction, but rather may be present out of solution (e.g., by virtue of having precipitated from the reaction). It is in a glucan synthesis reaction where the step of contacting water, sucrose and a glucosyltransferase enzyme is performed. The term "under suitable reaction conditions" as used herein refers to reaction conditions that support conversion of sucrose to alpha-glucan product(s) via glucosyltransferase enzyme activity.

The "yield" of an alpha-glucan product in a glucosyltransferase reaction in some aspects herein represents the molar yield based on the converted sucrose. The molar yield of an alpha-glucan product can be calculated based on the moles of the alpha-glucan product divided by the moles of the sucrose converted. Moles of converted sucrose can be calculated as follows: (mass of initial sucrose - mass of final sucrose) / molecular weight of sucrose [342 g/mol]. This molar yield calculation can be considered as a measure of selectivity of the reaction toward the alpha-glucan. In some aspects, the "yield" of an alpha-glucan product in a glucosyltransferase reaction can be based on the glucosyl component of the reaction. Such a yield (yield based on glucosyl) can be measured using the following formula:$Alpha - Glucan Yield = \left(IS/2-\left(FS/2+LE/2+GL+SO\right)\right) / \left(IS/2-FS/2\right) \times 100 % .$ The fructose balance of a glucosyltransferase reaction can be measured to ensure that HPLC data, if applicable, are not out of range (90-110% is considered acceptable). Fructose balance can be measured using the following formula:$Fructose Balance = \left(\left(180/342 x\left( FS+LE\right)+FR\right)/\left(180/342 x IS\right)\right) \times 100 % .$ In the above two formulae, IS is [Initial Sucrose], FS is [Final Sucrose], LE is [Leucrose], GL is [Glucose], SO is [Soluble Oligomers] (gluco-oligosaccharides), and FR is [Fructose]; the concentrations of each foregoing substrate/product provided in double brackets are in units of grams/L and as measured by HPLC, for example.

The terms "percent by volume", "volume percent", "vol %", "v/v %" and the like are used interchangeably herein. The percent by volume of a solute in a solution can be determined using the formula: [(volume of solute)/(volume of solution)] x 100%.

The terms "percent by weight", "weight percentage (wt%)", "weight-weight percentage (% w/w)" and the like are used interchangeably herein. Percent by weight refers to the percentage of a material on a mass basis as it is comprised in a composition, mixture, or solution.

The terms "aqueous conditions", "aqueous reaction conditions", "aqueous setting", "aqueous system" and the like are used interchangeably herein. Aqueous conditions herein refer to a solution or mixture in which the solvent is at least about 60 wt% water, for example. A glucosyltransferase reaction herein is performed under aqueous conditions.

The terms "soluble", "aqueous-soluble", "water-soluble" and the like as used herein characterize a glucan that has the capability of dissolving in water and/or an aqueous solution herein. Examples of soluble glucans herein are certain oligosaccharides, such as alpha-1,3-glucan with a DP less than 8. In contrast, a glucan that is "insoluble", "aqueous-insoluble", "water-insoluble" (and like terms) does not dissolve (or does not appreciably dissolve) in water and/or an aqueous solution herein. Optionally, the conditions for determining solubility include a water/solution temperature range of about 1 to 85 °C (e.g., 20-25 °C) and/or a pH range of about 4-9 (e.g., 6-8).

The terms "polynucleotide", "polynucleotide sequence", "nucleic acid molecule" and the like are used interchangeably herein. These terms encompass nucleotide sequences and the like. A polynucleotide may be a polymer of DNA or RNA that is single- or double-stranded, that optionally contains synthetic, non-natural or altered nucleotide bases. A polynucleotide may be comprised of one or more segments of cDNA, genomic DNA, synthetic DNA, or mixtures thereof.

The term "gene" as used herein refers to a DNA polynucleotide sequence that expresses an RNA (RNA is transcribed from the DNA polynucleotide sequence) from a coding region, which RNA can be a messenger RNA (encoding a protein) or a non-protein-coding RNA. A gene may refer to the coding region alone, or may include regulatory sequences upstream and/or downstream to the coding region (e.g., promoters, 5'-untranslated regions, 3'-transcription terminator regions). A coding region encoding a protein can alternatively be referred to herein as an "open reading frame" (ORF). A gene that is "native" or "endogenous" refers to a gene as found in nature with its own regulatory sequences; such a gene is located in its natural location in the genome of a host cell. A "chimeric" gene refers to any gene that is not a native gene, comprising regulatory and coding sequences that are not found together in nature (i.e., the regulatory and coding regions are heterologous with each other). Accordingly, a chimeric gene may comprise regulatory sequences and coding sequences that are derived from different sources, or regulatory sequences and coding sequences derived from the same source, but arranged in a manner different than that found in nature. A "foreign" or "heterologous" gene can refer to a gene that is introduced into the host organism by gene transfer. Foreign/heterologous genes can comprise native genes inserted into a non-native organism, native genes introduced into a new location within the native host, or chimeric genes. Polynucleotide sequences in certain embodiments disclosed herein are heterologous. A "transgene" is a gene that has been introduced into the genome by a gene delivery procedure (e.g., transformation). A "codon-optimized" open reading frame has its frequency of codon usage designed to mimic the frequency of preferred codon usage of the host cell.

As used herein, the term "polypeptide" is defined as a chain of amino acid residues, usually having a defined sequence. As used herein the term polypeptide is interchangeable with the terms "peptides" and "proteins". Typical amino acids contained in polypeptides herein include (respective three- and one-letter codes shown parenthetically): alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V).

The term "heterologous" means not naturally found in the location of interest. For example, a heterologous gene can be one that is not naturally found in a host organism, but that is introduced into the host organism by gene transfer. As another example, a nucleic acid molecule that is present in a chimeric gene can be characterized as being heterologous, as such a nucleic acid molecule is not naturally associated with the other segments of the chimeric gene (e.g., a promoter can be heterologous to a coding sequence).

A "non-native" amino acid sequence or polynucleotide sequence comprised in a cell or organism herein does not occur in a native (natural) counterpart of such cell or organism. Such an amino acid sequence or polynucleotide sequence can also be referred to as being heterologous to the cell or organism.

"Regulatory sequences" as used herein refer to nucleotide sequences located upstream of a gene's transcription start site (e.g., promoter), 5' untranslated regions, introns, and 3' non-coding regions, and which may influence the transcription, processing or stability, and/or translation of an RNA transcribed from the gene. Regulatory sequences herein may include promoters, enhancers, silencers, 5' untranslated leader sequences, introns, polyadenylation recognition sequences, RNA processing sites, effector binding sites, stem-loop structures, and other elements involved in regulation of gene expression. One or more regulatory elements herein may be heterologous to a coding region herein.

A "promoter" as used herein refers to a DNA sequence capable of controlling the transcription of RNA from a gene. In general, a promoter sequence is upstream of the transcription start site of a gene. Promoters may be derived in their entirety from a native gene, or be composed of different elements derived from different promoters found in nature, or even comprise synthetic DNA segments. Promoters that cause a gene to be expressed in a cell at most times under all circumstances are commonly referred to as "constitutive promoters". A promoter may alternatively be inducible. One or more promoters herein may be heterologous to a coding region herein.

A "strong promoter" as used herein refers to a promoter that can direct a relatively large number of productive initiations per unit time, and/or is a promoter driving a higher level of gene transcription than the average transcription level of the genes in a cell.

The terms "3' non-coding sequence", "transcription terminator", "terminator" and the like as used herein refer to DNA sequences located downstream of a coding sequence. This includes polyadenylation recognition sequences and other sequences encoding regulatory signals capable of affecting mRNA processing or gene expression.

As used herein, a first nucleic acid sequence is "hybridizable" to a second nucleic acid sequence when a single-stranded form of the first nucleic acid sequence can anneal to the second nucleic acid sequence under suitable annealing conditions (e.g., temperature, solution ionic strength). Hybridization and washing conditions are well known and exemplified in Sambrook J, Fritsch EF and Maniatis T, Molecular Cloning: A Laboratory Manual, 2nd ed., Cold Spring Harbor Laboratory: Cold Spring Harbor, NY (1989) , particularly Chapter 11 and Table 11.1.

The term "DNA manipulation technique" refers to any technique in which the sequence of a DNA polynucleotide sequence is modified. Although the DNA polynucleotide sequence being modified can be used as a substrate itself for modification, it does not have to be physically in hand for certain techniques (e.g., a sequence stored in a computer can be used as the basis for the manipulation technique). A DNA manipulation technique can be used to delete and/or mutate one or more DNA sequences in a longer sequence. Examples of a DNA manipulation technique include recombinant DNA techniques (restriction and ligation, molecular cloning), polymerase chain reaction (PCR), and synthetic DNA methods (e.g., oligonucleotide synthesis and ligation). Regarding synthetic DNA techniques, a DNA manipulation technique can entail observing a DNA polynucleotide *in silico,* determining desired modifications (e.g., one or more deletions) of the DNA polynucleotide, and synthesizing a DNA polynucleotide that contains the desired modifications.

The term *"in silico"* herein means in or on an information storage and/or processing device such as a computer; done or produced using computer software or simulation, i.e., virtual reality.

The terms "upstream" and "downstream" as used herein with respect to polynucleotides refer to "5' of" and "3' of", respectively.

The term "expression" as used herein refers to (i) transcription of RNA (e.g., mRNA or a non-protein-coding RNA) from a coding region, and/or (ii) translation of a polypeptide from mRNA. Expression of a coding region of a polynucleotide sequence can be up-regulated or down-regulated in certain embodiments.

The term "operably linked" as used herein refers to the association of two or more nucleic acid sequences such that the function of one is affected by the other. For example, a promoter is operably linked with a coding sequence when it is capable of affecting the expression of that coding sequence. That is, the coding sequence is under the transcriptional control of the promoter. A coding sequence can be operably linked to one (e.g., promoter) or more (e.g., promoter and terminator) regulatory sequences, for example.

The term "recombinant" when used herein to characterize a DNA sequence such as a plasmid, vector, or construct refers to an artificial combination of two otherwise separated segments of sequence, e.g., by chemical synthesis and/or by manipulation of isolated segments of nucleic acids by genetic engineering techniques.

The term "transformation" as used herein refers to the transfer of a nucleic acid molecule into a host organism or host cell by any method. A nucleic acid molecule that has been transformed into an organism/cell may be one that replicates autonomously in the organism/cell, or that integrates into the genome of the organism/cell, or that exists transiently in the cell without replicating or integrating. Non-limiting examples of nucleic acid molecules suitable for transformation are disclosed herein, such as plasmids and linear DNA molecules. Host organisms/cells herein containing a transforming nucleic acid sequence can be referred to as "transgenic", "recombinant", "transformed", "engineered", as a "transformant", and/or as being "modified for exogenous gene expression", for example.

The terms "sequence identity", "identity" and the like as used herein with respect to polynucleotide or polypeptide sequences refer to the nucleic acid residues or amino acid residues in two sequences that are the same when aligned for maximum correspondence over a specified comparison window. Thus, "percentage of sequence identity", "percent identity" and the like refer to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. It would be understood that, when calculating sequence identity between a DNA sequence and an RNA sequence, T residues of the DNA sequence align with, and can be considered "identical" with, U residues of the RNA sequence. For purposes of determining "percent complementarity" of first and second polynucleotides, one can obtain this by determining (i) the percent identity between the first polynucleotide and the complement sequence of the second polynucleotide (or vice versa), for example, and/or (ii) the percentage of bases between the first and second polynucleotides that would create canonical Watson and Crick base pairs.

Percent identity can be readily determined by any known method, including but not limited to those described in: 1) Computational Molecular Biology (Lesk, A.M., Ed.) Oxford University: NY (1988); 2) Biocomputing: Informatics and Genome Projects (Smith, D.W., Ed.) Academic: NY (1993); 3) Computer Analysis of Sequence Data, Part I (Griffin, A.M., and Griffin, H.G., Eds.) Humana: NJ (1994); 4) Sequence Analysis in Molecular Biology (von Heinje, G., Ed.) Academic (1987); and 5) Sequence Analysis Primer (Gribskov, M. and Devereux, J., Eds.) Stockton: NY (1991).

Preferred methods for determining percent identity are designed to give the best match between the sequences tested. Methods of determining identity and similarity are codified in publicly available computer programs, for example. Sequence alignments and percent identity calculations can be performed using the MEGALIGN program of the LASERGENE bioinformatics computing suite (DNASTAR Inc., Madison, WI), for example. Multiple alignment of sequences can be performed, for example, using the Clustal method of alignment which encompasses several varieties of the algorithm including the Clustal V method of alignment (described by Higgins and Sharp, CABIOS. 5:151-153 (1989); Higgins, D.G. et al., Comput. Appl. Biosci., 8:189-191 (1992)) and found in the MEGALIGN v8.0 program of the LASERGENE bioinformatics computing suite (DNASTAR Inc.). For multiple alignments, the default values can correspond to GAP PENALTY=10 and GAP LENGTH PENALTY=10. Default parameters for pairwise alignments and calculation of percent identity of protein sequences using the Clustal method can be KTUPLE=1, GAP PENALTY=3, WINDOW=5 and DIAGONALS SAVED=5. For nucleic acids, these parameters can be KTUPLE=2, GAP PENALTY=5, WINDOW=4 and DIAGONALS SAVED=4. Additionally, the Clustal W method of alignment can be used (described by Higgins and Sharp, CABIOS. 5:151-153 (1989); Higgins, D.G. et al., Comput. Appl. Biosci. 8:189-191(1992); Thompson, J.D. et al, Nucleic Acids Research, 22 (22): 4673-4680, 1994) and found in the MEGALIGN v8.0 program of the LASERGENE bioinformatics computing suite (DNASTAR Inc.). Default parameters for multiple alignment (protein/nucleic acid) can be: GAP PENALTY=10/15, GAP LENGTH PENALTY=0.2/6.66, Delay Divergent Seqs(%)=30/30, DNA Transition Weight=0.5, Protein Weight Matrix=Gonnet Series, DNA Weight Matrix=IUB.

Various polypeptide amino acid sequences and polynucleotide sequences are disclosed herein as features of certain embodiments. Variants of these sequences that are at least about 70-85%, 85-90%, or 90%-95% identical to the sequences disclosed herein can be used or referenced. Alternatively, a variant amino acid sequence or polynucleotide sequence can have at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with a sequence disclosed herein. The variant amino acid sequence or polynucleotide sequence has the same function/activity of the disclosed sequence, or at least about 80%, 81%, 82%, 83%, 84%, 85%, 86%, 87%, 88%, 89%, 90%, 91%, 92%, 93%, 94%, 95%, 96%, 97%, 98%, or 99% of the function/activity of the disclosed sequence. Any polypeptide amino acid sequence disclosed herein not beginning with a methionine can typically further comprise at least a start-methionine at the N-terminus of the amino acid sequence. In contrast, any polypeptide amino acid sequence disclosed herein beginning with a methionine can optionally lack such a methionine residue.

The terms "aligns with", "corresponds with", and the like can be used interchangeably herein. An amino acid position of a glucosyltransferase or subsequence thereof (e.g., catalytic domain or catalytic domain plus glucan-binding domains) (can refer to such an amino acid position or sequence as a "query" position or sequence) can be characterized to correspond with a particular amino acid residue of SEQ ID NO:62 (can refer to such an amino acid position or sequence as a "subject" position or sequence) if (1) the query sequence can be aligned with the subject sequence (e.g., where an alignment indicates that the query sequence and the subject sequence [or a subsequence of the subject sequence] are at least about 30%, 40%, 50%, 60%, 70%, 80%, or 90% identical), and (2) if the query amino acid position directly aligns with (directly lines up against) the subject amino acid position in the alignment of (1). In general, one can align a query amino acid sequence with a subject sequence (SEQ ID NO:62 or a subsequence of SEQ ID NO:62) using any alignment algorithm, tool and/or software described disclosed herein (e.g., BLASTP, ClustalW, ClustalV, Clustal-Omega, EMBOSS) to determine percent identity. Just for further example, one can align a query sequence with a subject sequence herein using the Needleman-Wunsch algorithm (Needleman and Wunsch, J. Mol. Biol. 48:443-453, 1970) as implemented in the Needle program of the European Molecular Biology Open Software Suite (EMBOSS [e.g., version 5.0.0 or later], Rice et al., Trends Genet. 16:276-277, 2000). The parameters of such an EMBOSS alignment can comprise, for example: gap open penalty of 10, gap extension penalty of 0.5, EBLOSUM62 (EMBOSS version of BLOSUM62) substitution matrix.

The numbering of particular amino acid residues of SEQ ID NO:62 herein (e.g., Leu-373, Leu-428, Ala-472, Ala-510, Leu-513, Met-529, Gln-588, Phe-607, Asn-613, Gln-616, Ser-631, Gly-633, Phe-634, Ser-710, Thr-635, Arg-722, Arg-741, Thr-877, Asp-948, Phe-951, Gln-957, Val-1188, Met-1253) is with respect to the full-length amino acid sequence of SEQ ID NO:62. The first amino acid (i.e., position 1, Met-1) of SEQ ID NO:62 is at the start of the signal peptide. Unless otherwise disclosed, substitutions herein are with respect to the full-length amino acid sequence of SEQ ID NO:62.

A "non-native glucosyltransferase" herein ("mutant", "variant", "modified" and like terms can likewise be used to describe such a glucosyltransferase) has at least one amino acid substitution at a position corresponding with a particular amino acid residue of SEQ ID NO:62. Such at least one amino acid substitution typically is in place of the amino acid residue(s) that normally (natively) occurs at the same position in the native counterpart (parent) of the non-native glucosyltransferase (i.e., although SEQ ID NO:62 is used as a reference for position, an amino acid substitution herein is with respect to the native counterpart of a non-native glucosyltransferase) (considered another way, when aligning the sequence of a non-native glucosyltransferase with SEQ ID NO:62, determining whether a substitution exists at a particular position does not depend in- and-of-itself on the respective amino acid residue in SEQ ID NO:62, but rather depends on what amino acid exists at the subject position within the native counterpart of the non-native glucosyltransferase). The amino acid normally occurring at the relevant site in the native counterpart glucosyltransferase often (but not always) is the same as (or conserved with) the particular amino acid residue of SEQ ID NO:62 for which the alignment is made. A non-native glucosyltransferase optionally can have other amino acid changes (mutations, deletions, and/or insertions) relative to its native counterpart sequence.

It may be instructive to illustrate a substitution/alignment herein. SEQ ID NO:12 (GTF 0544) is a truncated form of a *Streptococcus sobrinus* glucosyltransferase. It is noted that Leu-193 of SEQ ID NO:12 corresponds with Leu-373 of SEQ ID NO:62 (alignment not shown). If SEQ ID NO:12 is mutated at position 193 to substitute the Leu residue with a different residue (e.g., Gln), then it can be stated that the position 193-mutated version of SEQ ID NO:12 represents a non-native glucosyltransferase having an amino acid substitution at a position corresponding with Leu-373 of SEQ ID NO:62, for example.

The term "isolated" means a substance (or process) in a form or environment that does not occur in nature. Non-limiting examples of isolated substances include (1) any non-naturally occurring substance (e.g., a non-native glucosyltransferase herein), (2) any substance including, but not limited to, any host cell, enzyme, variant, nucleic acid, protein, peptide, cofactor, or carbohydrate/saccharide that is at least partially removed from one or more or all of the naturally occurring constituents with which it is associated in nature; (3) any substance modified by the hand of man relative to that substance found in nature (e.g., a non-native glucosyltransferase herein); or (4) any substance modified by increasing the amount of the substance relative to other components with which it is naturally associated. It is believed that the embodiments (e.g., enzymes and reaction compositions) disclosed herein are synthetic/man-made, and/or have properties that are not naturally occurring.

The term "increased" as used herein can refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", "improved" and the like are used interchangeably herein. These terms can be used to characterize the "over-expression" or "upregulation" of a polynucleotide encoding a protein, for example.

While advances have been made in producing glucan polymers using glucosyltransferase enzymes, less attention appears to have been drawn to improving the glucan yields of such enzymes. Addressing this technological gap, disclosed herein are glucosyltransferases engineered to have modified amino acid sequences endowing these enzymes with enhanced glucan production properties.

The present disclosure concerns a non-native glucosyltransferase comprising at least two or three amino acid substitutions at positions corresponding with amino acid residues Gln-588, Phe-607, and/or Arg-741 of SEQ ID NO:62, wherein the non-native glucosyltransferase synthesizes alpha-glucan comprising 1,3-linkages, and wherein the non-native glucosyltransferase has:
(i) an alpha-glucan yield that is higher than the alpha-glucan yield of a second glucosyltransferase that only differs from the non-native glucosyltransferase at the substitution position(s), and/or
(ii) a leucrose yield that is lower than the leucrose yield of the second glucosyltransferase;
   wherein the glucosyltransferase comprises a catalytic domain that is at least 90% identical to residues 55-960 of SEQ ID NO:4; and
   wherein:
      (a) the amino acid substitution at the position corresponding with amino acid residue Gln-588 is with a Leu, Ala, or Val residue;
      (b) the amino acid substitution at the position corresponding with amino acid residue Phe-607 is with a Trp or Asn residue; and/or
      (c) the amino acid substitution at the position corresponding with amino acid residue Arg-741 is with a Ser or Thr residue.
   Thus, in general, mutant glucosyltransferase enzymes are disclosed herein that can synthesize higher amounts of alpha-glucan, and/or lower yields of leucrose, which is a by-product often considered undesirable when the main goal is alpha-glucan synthesis.

A non-native glucosyltransferase herein synthesizes alpha-glucan comprising 1,3-linkages. In some aspects, at least about 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 69%, 70%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or 100% of the glycosidic linkages of such an alpha-glucan can be alpha-1,3 linkages. The linkage profile of an alpha-glucan can optionally be characterized as having a range between any two of these values. The other linkages in any of these aspects having 30%-99% alpha-1,3 linkages can be alpha-1,6, and/or not include any alpha-1,4 or alpha-1,2 linkages, for example.

Alpha-glucan in some aspects can have, for example, less than 10%, 9%, 8%, 7%, 6%, 5%, 4%, 3%, 2%, 1%, or 0.5% of alpha-1,2 or alpha-1,4 glycosidic linkages. In another embodiment, an alpha-glucan only has alpha-1,3 and optionally alpha-1,6 linkages (i.e., no alpha-1,2 or alpha-1,4 linkages).

Alpha-glucan in some aspects can be linear/unbranched (no branch points). Alternatively, there can be branches in an alpha-glucan herein. For example, an alpha-glucan can have less than about 10%, 9%, 8%, 7%, 6%, 5%, 4%, 3%, 2%, or 1% branch points as a percent of the linkages in the polymer.

In certain aspects, an alpha-glucan can have a molecular weight in DP_{w} or DPₙ of at least about 100. For example, the DP_{w} or DPₙ can be about, or at least about, 100, 150, 200, 250, 300, 350, 400, 450, 500, 550, 600, 650, 700, 750, 800, 850, 900, 950, 1000, 1100, or 1200. The molecular weight of an alpha-glucan can optionally be expressed as a range between any two of these values (e.g., 100-1200, 400-1200, 700-1200, 100-1000, 400-1000, 700-1000).

An alpha-glucan produced by a non-native glucosyltransferase herein typically is water-insoluble. Alpha-1,3-glucan is generally insoluble at a DP_{w} of 8 or 9 and above in neutral (e.g., pH 6-8) aqueous conditions.

Any of the foregoing linkage profiles and/or molecular weight profiles, for example, can be combined herein to appropriately characterize an alpha-glucan product of a non-native glucosyltransferase of the present disclosure. In some aspects, the linkage and/or molecular weight profile of an alpha-glucan product can be as disclosed in any of the following publications: U.S. Patent Nos. 7000000 and 8871474, U.S. Patent Appl. Publ. No. 2015/0232819.

A non-native glucosyltransferase, for example, can comprise the amino acid sequence of any glucosyltransferase disclosed in the following publications that is capable of producing alpha-glucan as presently disclosed, but with the exception that the non-native glucosyltransferase comprises at least two of, or all three of, amino acid substitutions at positions corresponding with amino acid residues Gln-588, Phe-607, and/or Arg-741 of SEQ ID NO:62: U.S. Patent Nos. 7000000 and 8871474; and U.S. Patent Appl. Publ. Nos. 2015/0232819 and 2017/0002335. In some aspects, such a non-native glucosyltransferase (i) comprises the foregoing substitutions, and (ii) comprises an amino acid sequence that is at least about 90%, 91%, 92%, 93%, 94%, 95%, 96%, 97%, 98%, 99%, or 99.5% identical to the amino acid sequence of the respective counterpart/parent glucosyltransferase not having the foregoing substitutions.

Certain information regarding alpha-glucan products of glucosyltransferases with most of these amino acid sequences is provided in Table 2.

**Table 2. GTF Enzymes and Related Alpha-Glucan Products^{a}**

| GTF ID | SEQ ID NO. | Reducing Sugars | Insoluble Product | Linkages | | DPₙ |
|---|---|---|---|---|---|---|
| | | | | % alpha-1,3 | % alpha-1,6 | |
| 0874 | 2 | yes | yes | 100 | 0 | 60 |
| 6855 | 4 | yes | yes | 100 | 0 | 440 |
| 2379 | 6 | yes | yes | 37 | 63 | 310 |
| 7527 | 8 | yes | yes | 100 | 0 | 440 |
| 1724 | 10 | yes | yes | 100 | 0 | 250 |
| 0544 | 12 | yes | yes | 62 | 36 | 980 |
| 5926 | 14 | yes | yes | 100 | 0 | 260 |
| 4297 | 16 | yes | yes | 31 | 67 | 800 |
| 5618 | 18 | yes | yes | 34 | 66 | 1020 |
| 2765 | 20 | yes | yes | 100 | 0 | 280 |
| 0427 | 26 | yes | yes | 100 | 0 | 120 |
| 2919 | 28 | yes | yes | 100 | 0 | 250 |
| 2678 | 30 | yes | yes | 100 | 0 | 390 |
| 3929 | 34 | yes | yes | 100 | 0 | 280 |

| | | | | | | |
|---|---|---|---|---|---|---|
| ^{a} GTF reactions and product analyses were performed as follows. Reactions were prepared comprising sucrose (50 g/L), potassium phosphate buffer (pH 6.5, 20 mM) and a GTF enzyme (2.5% bacterial cell extract by volume; extracts prepared according to U.S. Appl. Publ. No. 2017/0002335, in a manner similar to procedure disclosed in U.S. Patent No. 8871474). After 24-30 hours at 22-25 °C, insoluble product was harvested by centrifugation, washed three times with water, washed once with ethanol, and dried at 50 °C for 24-30 hours. Approximate linkages and DPₙ are shown for each insoluble product. Linkages and DPₙ were determined by ¹³C NMR and SEC, respectively. | | | | | | |

In some aspects, a non-native glucosyltransferase comprises or consists of a glucosyltransferase catalytic domain that is at least about 90%, 91%, 92%, 93%, 94%, 95%, 96%, 97%, 98%, 99%, or 99.5% identical to amino acid residues 55-960 of SEQ ID NO:4. Such a non-native glucosyltransferase, for instance, is believed to be able to produce alpha-glucan that is water-insoluble and comprise at least about 50% (e.g., ≥90% or ≥95%) alpha-1,3 linkages, and optionally further have a DP_{w} of at least 100. It is noted that a glucosyltransferase with amino acid positions 54-957 of SEQ ID NO:65 can produce alpha-1,3-glucan with 100% alpha-1,3 linkages and a DP_{w} of at least 400 (data not shown, refer to Table 6 of U.S. Pat. Appl. Publ. No. 2017/0002335), for example. It is further noted that SEQ ID NOs:65 (GTF 7527), 30 (GTF 2678), 4 (GTF 6855), 28 (GTF 2919), and 20 (GTF 2765) each represent a glucosyltransferase that, compared to its respective wild type counterpart, lacks the signal peptide domain and all or a substantial portion of the variable domain. Thus, each of these glucosyltransferase enzymes has a catalytic domain followed by a glucan-binding domain. The approximate location of catalytic domain sequences in these enzymes is as follows: 7527 (residues 54-957 of SEQ ID NO:65), 2678 (residues 55-960 of SEQ ID NO:30), 6855 (residues 55-960 of SEQ ID NO:4), 2919 (residues 55-960 of SEQ ID NO:28), 2765 (residues 55-960 of SEQ ID NO:20). The amino acid sequences of the catalytic domains (approx.) of GTFs 2678, 6855, 2919 and 2765 have about 94.9%, 99.0%, 95.5% and 96.4% identity, respectively, with the approximate catalytic domain sequence of GTF 7527 (i.e., amino acids 54-957 of SEQ ID NO:65). Each of these particular glucosyltransferases (GTFs 2678, 6855, 2919 and 2765) can produce alpha-1,3-glucan with 100% alpha-1,3 linkages and a DP_{w} of at least 400 (data not shown, refer to Table 4 of U.S. Pat. Appl. Publ. No. 2017/0002335). Based on this activity, and the relatedness (high percent identity) of the foregoing catalytic domains, it is contemplated that a non-native glucosyltransferase herein having one of the foregoing catalytic domains further with an amino acid substitution combination as presently disclosed can produce alpha-glucan comprising at least about 50% (e.g., ≥ 90% or ≥95%) alpha-1,3 linkages and a DP_{w} of at least 100.
).

Although it is believed that a non-native glucosyltransferase in certain aspects need only have a catalytic domain, the non-native glucosyltransferase can be comprised within a larger amino acid sequence. For example, a catalytic domain may be linked at its C-terminus to a glucan-binding domain, and/or linked at its N-terminus to a variable domain and/or signal peptide.

Although amino acid substitutions in a non-native glucosyltransferase are generally disclosed herein with respect to the corresponding positions in SEQ ID NO:62, such substitutions can alternatively be stated simply with respect to its position number in the sequence of the non-native glucosyltransferase itself, as convenience may dictate.

Still further examples of non-native glucosyltransferases can be any as disclosed herein and that include 1-300 (or any integer there between [e.g., 10, 15, 20, 25, 30, 35, 40, 45, or 50]) residues on the N-terminus and/or C-terminus. Such additional residues may be from a corresponding wild type sequence from which the glucosyltransferase enzyme is derived, or may be a heterologous sequence such as an epitope tag (at either N- or C-terminus) or a heterologous signal peptide (at N-terminus), for example. A non-native glucosyltransferase herein typically lacks an N-terminal signal peptide; such an enzyme can optionally be characterized as being mature if its signal peptide was removed during a secretion process.

A non-native glucosyltransferase herein can be derived from any microbial source, for example, such as bacteria. Examples of bacterial glucosyltransferases are those derived from a *Streptococcus* species, *Leuconostoc* species, or *Lactobacillus* species. Examples of *Streptococcus* species include *S*. *salivarius, S. sobrinus, S. dentirousetti, S. downei, S. mutans, S*. *oralis, S. gallolyticus* and *S*. *sanguinis.* Examples of *Leuconostoc* species include *L. mesenteroides, L. amelibiosum, L. argentinum, L. carnosum, L. citreum, L. cremoris, L. dextranicum* and *L. fructosum.* Examples of *Lactobacillus* species include *L. acidophilus, L. delbrueckii, L. helveticus, L. salivarius, L. casei, L. curvatus, L. plantarum, L. sakei, L. brevis, L. buchneri, L. fermentum* and *L*. *reuteri.*

A non-native glucosyltransferase herein can be prepared by fermentation of an appropriately engineered microbial strain, for example. Recombinant enzyme production by fermentation is well known in the art using microbial species such as *E*. *coli, Bacillus* strains (e.g., *B. subtilis), Ralstonia eutropha, Pseudomonas fluorescens, Saccharomyces cerevisiae, Pichia pastoris, Hansenula polymorpha,* and species of *Aspergillus* (e.g., *A. awamori)* and *Trichoderma* (e.g., *T. reesei*) (e.g., see Adrio and Demain, Biomolecules 4:117-139, 2014). A nucleotide sequence encoding a non-native glucosyltransferase amino acid sequence is typically linked to a heterologous promoter sequence to create an expression cassette for the enzyme, and/or is codon-optimized accordingly. Such an expression cassette may be incorporated in a suitable plasmid or integrated into the microbial host chromosome, using methods well known in the art. The expression cassette may include a transcriptional terminator nucleotide sequence following the amino acid coding sequence. The expression cassette may also include, between the promoter sequence and glucosyltransferase amino acid coding sequence, a nucleotide sequence encoding a signal peptide (e.g., heterologous signal peptide) that is designed for direct secretion of the glucosyltransferase enzyme. At the end of fermentation, cells may be ruptured accordingly (generally when a signal peptide for secretion is not employed) and the glucosyltransferase enzyme can be isolated using methods such as precipitation, filtration, and/or concentration. Alternatively, a lysate or extract comprising a glucosyltransferase can be used without further isolation. If the glucosyltransferase was secreted (i.e., it is present in the fermentation broth), it can optionally be used as isolated from, or as comprised in, the fermentation broth. The activity of a glucosyltransferase enzyme can be confirmed by biochemical assay, such as measuring its conversion of sucrose to glucan polymer.

A non-native glucosyltransferase herein comprises amino acid substitutions at positions corresponding with at least two of, or all three of, amino acid residues Gln-588, Phe-607 and/or Arg-741 of SEQ ID NO:62.The amino acid substitution at a position corresponding with amino acid Gln-588 of SEQ ID NO:62 can be with a Leu, Ala, or Val residue. The amino acid substitution at a position corresponding with amino acid Phe-607 of SEQ ID NO:62 can be with a Trp or Asn residue. The amino acid substitution at a position corresponding with amino acid Arg-741 of SEQ ID NO:62 can be with a Ser or Thr residue. Examples of a non-native glucosyltransferase herein comprise at least: (A) Gln-588-Leu, Phe-607-Trp and Arg-741-Ser substitutions. In some aspects, a non-native glucosyltransferase herein can comprise amino acid substitutions at positions corresponding with amino acid residues (i) Gln-588 and Phe-607, (ii) Gln-588 and Arg-741, or (iii) Phe-607 and Arg-741 of SEQ ID NO:62.

A non-native glucosyltransferase herein can comprise, in addition to the foregoing two or three amino acid substitutions, one, two, three, four, five, six, seven, eight, nine, or more of the disclosed amino acid substitutions, for instance. For example, a non-native glucosyltransferase can further comprise at least one amino acid substitution at a position corresponding with amino acid residue Ala-510 and/or Asp-948 of SEQ ID NO:62. In some aspects, the amino acid substitution at a position corresponding with amino acid Ala-510 of SEQ ID NO:62 can be with an Asp, Glu, Ile, or Val residue. In some aspects, the amino acid substitution at a position corresponding with amino acid Asp-948 of SEQ ID NO:62 can be with a Gly, Val, or Ala residue. Examples of a non-native glucosyltransferase herein comprise at least: (B) Ala-510-Glu and Asp-948-Val substitutions; (C) an Asp-948-Ala substitution; or (D) Ala-510-Asp and Asp-948-Gly substitutions, (in addition to the foregoing substitution combination of A).

In another example, a non-native glucosyltransferase can further comprise at least one amino acid substitution at a position corresponding with amino acid residue Ser-631, Ser-710, Arg-722, and/or Thr-877 of SEQ ID NO:62. In some aspects, the amino acid substitution at a position corresponding with amino acid Ser-631 of SEQ ID NO:62 can be with a Thr, Asp, Glu, or Arg residue. In some aspects, the amino acid substitution at a position corresponding with amino acid Ser-710 of SEQ ID NO:62 can be with a Gly, Ala, or Val residue. In some aspects, the amino acid substitution at a position corresponding with amino acid Arg-722 of SEQ ID NO:62 can be with a His or Lys residue. In some aspects, the amino acid substitution at a position corresponding with amino acid Thr-877 of SEQ ID NO:62 can be with a Lys, His, or Arg residue. Examples of a non-native glucosyltransferase herein comprise at least: (E) Ser-631-Thr, Ser-710-Gly, Arg-722-His and Thr-877-Lys substitutions; (F) Ser-710-Ala, Arg-722-Lys and Thr-877-Lys substitutions; or (G) Ser-631-Ser, Ser-710-Gly, and Thr-877-Arg substitutions, (in addition to any of the foregoing substitution combinations of [i] A with B, C, or D).

In another example, a non-native glucosyltransferase can further comprise at least one amino acid substitution at a position corresponding with amino acid residue Val-1188, Met-1253, and/or Gln-957 of SEQ ID NO:62. In some aspects, the amino acid substitution at a position corresponding with amino acid Val-1188 of SEQ ID NO:62 can be with a Glu or Asp residue. In some aspects, the amino acid substitution at a position corresponding with amino acid Met-1253 of SEQ ID NO:62 can be with an Ile, Leu, Ala, or Val residue. In some aspects, the amino acid substitution at a position corresponding with amino acid Gln-957 of SEQ ID NO:62 can be with a Pro residue. Examples of a non-native glucosyltransferase herein comprise at least: (H) a Val-1188-Asp substitution; (I) a Met-1253-Ile substitution; (J) Val-1188-Glu and Met-1253-Ile substitutions; (K) Val-1188-Glu, Met-1253-Ile and Gln-957-Pro substitutions; or (L) a Val-1188-Glu substitution (in addition to any of the foregoing substitution combinations of [i] A with B, C, or D; [ii] A with E, F, or G; [ill] A with one of B, C, or D, and one of E, F, or G).

Other suitable substitutions that can be in addition to those listed above, for example, include those as listed in Table 3 in Example 1 (below) that are associated with (i) a decrease in leucrose production by at least about 5%, 10%, 15%, 20%, 25%, 30%, 35%, 40%, 45%, 50%, 55%, 60%, 65%, 70%, 75%, 80%, 85%, 90%, or 95%, and/or (ii) an increase in glucan yield by at least about 5%, 10%, 15%, 20%, 25%, 30%, 35%, 40%, 45%, 50%, 55%, 60%, 65%, 70%, 75%, 80%, 85%, 90%, 95%, 100%, 105%, 110%, 115%, 120%, 125%, 130%, 135%, 140%, 145%, or 150%. In some aspects, suitable additional substitutions include those as listed in Table 3 in Example 1 (below) that are associated with a decrease in glucose production by at least about 5%, 10%, 15%, 20%, 25%, 30%, 35%, 40%, 45%, 50%, 55%, 60%, 65%, 70%, 75%, or 80%. In some aspects, suitable additional substitutions include those as listed in Table 3 in Example 1 (below) that are associated with a decrease in gluco-oligosaccharide (oligomer) production by at least about 5%, 10%, 15%, 20%, 25%, 30%, 35%, 40%, 45%, 50%, 55%, 60%, or 65%. The foregoing substitutions as listed in Table 3 are as they correspond with the listed residue position number in SEQ ID NO:62. In some aspects, one or more substitutions are conserved or non-conserved substitutions; such conservation (or not) can be, for instance, with respect to the amino acid that occurs in the native glucosyltransferase from which the non-native glucosyltransferase is derived.

Simply for illustration purposes, a non-native glucosyltransferase herein can comprise a combination of amino acid substitutions at positions as follows (i-xix), where each substitution position corresponds with the respective amino acid position number of SEQ ID NO:62:
(i) A510, Q588, F607, R741, D948, R722, T877, M1253 and K1277;
(ii) A510, Q588, F607, R741, D948, R722, T877, V1188, M1253 and Q957;
(iii) A510, Q588, F607, R741, D948, T877, V1188, M1253 and Q957;
(iv) A510, Q588, F607, R741, D948 and M1253;
(v) A510, Q588, F607, R741 and D948;
(vi) Q588, F607, R741 and D948;
(vii) A510, Q588, F607, R741, D948, N628, T635, T877, M1253, F929 and R1172;
(viii) A510, Q588, F607, R741, D948, S631, S710, R722, T877, V1188 and M1253;
(ix) A510, Q588, F607, R741, D948, S631, S710, R722, T877 and V1188;
(x) A510, Q588, F607, R741, D948, S631, S710, T877, V1188 and M1253;
(xi) A510, Q588, F607, R741 and D948;
(xii) A510, Q588, F607, R741, D948 and V1188;
(xiii) A510, Q588, F607, R741, D948, S631, S710 and V1188;
(xiv) A510, Q588, F607, R741, D948, S710, R722, T877 and M1253;
(xv) A510, Q588, F607, R741, D948, S631, R722, T877, V1188 and M1253;
(xvi) A510, Q588, F607, R741, D948, S631, T877, V1188 and M1253;
(xvii) A510, Q588, F607, R741, D948, S631 and V1188;
(xviii) A510, Q588, F607, R741, D948, S631, R722, T877, V1188 and M1253; or
(xix) A510, Q588, F607, R741, D948, V1188 and M1253;
   Some particular examples of embodiments i-xix are disclosed in Example 4 below (Table 7). Thus, a non-native glucosyltransferase in some aspects can comprise one of the following combinations of substitutions (xx-xxviii), where each substitution corresponds with the respective amino acid residue of SEQ ID NO:62:
(xx) A510D/Q588L/F607W/R741S/D948G,
(xxi)A510D/Q588L/F607W/R741S/D948G/S631T/S710G/R722H/T877K/V1188E/M1253,
(xxii) A510D/Q588L/F607W/R741S/D948G/S631T/S710G/R722H/T877K/V1188E,
(xxiii) A510D/Q588L/F607W/R741S/D948G/S631T/S710G/T877K/V1188E/M12531,
(xxiv) A510D/Q588L/F607W/R741S/D948G/S631T/S710G/V1188E,
(xxv) A510D/Q588L/F607W/R741S/D948G/S710G/R722H/T877K/M1253I,
(xxvi) A510D/Q588L/F607W/R741S/D948G/S631T/T877K/V1188E/M1253I,
(xxvii) A510D/Q588L/F607W/R741S/D948G/S631T/V1188E,
   or
(xxviii) A510D/Q588L/F607W/R741S/D948G/V1188E/M1253I.

A non-native glucosyltransferase with a combination of amino acid substitutions herein can be based on any of a variety of glucosyltransferase amino acid sequences as presently disclosed, for example. Simply for illustration purposes, examples of such a non-native glucosyltransferase include those with a combination of amino acid substitutions as described herein (e.g., any of embodiments i-xxviii above)

A non-native glucosyltransferase herein has (i) an alpha-glucan yield that is higher than the alpha-glucan yield of a second glucosyltransferase (or, simply, "another" glucosyltransferase) (e.g., parent glucosyltransferase) that only differs from the non-native glucosyltransferase at the substitution positions, and/or (ii) a leucrose yield that is lower than the leucrose yield of the second glucosyltransferase. A second glucosyltransferase herein, for example, can be comprised of all of, or mostly, native amino acid sequence. Thus, while a second glucosyltransferase herein can be a native glucosyltransferase in some aspects, it can be a prior-modified glucosyltransferase in other aspects (e.g., a glucosyltransferase with one or more other amino acid substitutions differing from the substitution[s] of the present disclosure). In some embodiments, a second glucosyltransferase to which a non-native glucosyltransferase is compared has native amino acid residues at the substitution positions. Determining whether an amino acid residue is native can be done by comparing the second glucosyltransferase amino acid sequence to the native/wild type glucosyltransferase amino acid sequence from which the second glucosyltransferase is derived. Optionally, a non-native glucosyltransferase in some embodiments can be characterized as having higher selectivity toward alpha-glucan synthesis (as compared to by-product synthesis).

In some aspects, a non-native glucosyltransferase herein can have an alpha-glucan yield that is at least about 5%, 10%, 20%, 40%, 60%, 80%, 100%, 120%, 140%, 160%, 180%, 200%, 220%, 240%, 260%, 280%, 300%, 320%, 340%, 360%, or 380% higher than the alpha-glucan yield of a second glucosyltransferase as presently disclosed. In some additional or alternative embodiments, a non-native glucosyltransferase can have a decrease in leucrose yield by at least about 5%, 10%, 15%, 20%, 25%, 30%, 35%, 40%, 45%, 50%, 55%, 60%, 65%, 70%, 75%, 80%, 85%, 90%, or 95% compared to the leucrose yield of a second glucosyltransferase. These determinations (alpha-glucan and/or leucrose yield) can be made with respect to any glucan synthesis reaction/process as disclosed herein (e.g., taking into account initial sucrose conc., temperature, pH, and/or reaction time), and using any suitable measurement technique (e.g., HPLC or NIR spectroscopy). Typically, a comparison between non-native and second glucosyltransferases herein can be made under identical or similar reaction conditions. The yield of a glucosyltransferase reaction in some aspects can be measured based on the glucosyl component of the reaction.

In some embodiments, a non-native glucosyltransferase can exhibit a decrease in the yield of soluble gluco-oligosaccharides by at least about 5%, 10%,15%, 20%, 25%, 30%, 35%, 40%, 45%, 50%, 55%, 60%, 65%, 70%, or 75% compared to the soluble gluco-oligosaccharide yield of a second glucosyltransferase. A soluble gluco-oligosaccharide in some aspects can be DP2-7 or DP2-8, and have any linkage profile disclosed herein. In some aspects, the DP is ≥7, or up to 10, 15, 20, or 25, but with a linkage profile allowing solubility (e.g., not over 90% or 95% alpha-1,3).

In some embodiments, a non-native glucosyltransferase can exhibit a decrease in the yield of glucose by at least about 5%, 10%,15%, 20%, 25%, 30%, 35%, 40%, 45%, 50%, 55%, 60%, 65%, 70%, 75%, or 80% compared to the glucose yield of a second glucosyltransferase.

Some embodiments disclosed herein concern a polynucleotide comprising a nucleotide sequence that encodes a non-native glucosyltransferase as presently disclosed, wherein one or more regulatory sequences are operably linked to the nucleotide sequence, and preferably wherein a promoter sequence is included as a regulatory sequence.

A polynucleotide comprising a nucleotide sequence encoding a non-native glucosyltransferase herein can be a vector or construct useful for transferring a nucleotide sequence into a cell, for example. Examples of a suitable vector/construct can be selected from a plasmid, yeast artificial chromosome (YAC), cosmid, phagemid, bacterial artificial chromosome (BAC), virus, or linear DNA (e.g., linear PCR product). A polynucleotide sequence in some aspects can be capable of existing transiently (i.e., not integrated into the genome) or stably (i.e., integrated into the genome) in a cell. A polynucleotide sequence in some aspects can comprise, or lack, one or more suitable marker sequences (e.g., selection or phenotype marker).

A polynucleotide sequence comprises one or more regulatory sequences operably linked to the nucleotide sequence encoding a non-native glucosyltransferase. For example, a nucleotide sequence encoding a non-native glucosyltransferase may be in operable linkage with a promoter sequence (e.g., a heterologous promoter). A promoter sequence can be suitable for expression in a cell (e.g., bacterial cell such as E. *coli* or *Bacillus*; eukaryotic cell such as a fungus, yeast, insect, or mammalian cell) or in an *in vitro* protein expression system, for example. Examples of other suitable regulatory sequences include transcription terminator sequences.

Some aspects herein are drawn to a cell comprising a polynucleotide sequence as presently disclosed; such a cell can be any type disclosed herein (e.g., bacterial cell such as *E. coli* or *Bacillus*; eukaryotic cell such as a fungus, yeast, insect, or mammalian cell). A cell can optionally express a non-native glucosyltransferase encoded by the polynucleotide sequence. In some aspects, the polynucleotide sequence exists transiently (i.e., not integrated into the genome) or stably (i.e., integrated into the genome) in the cell.

Some embodiments disclosed herein concern reaction compositions comprising water, sucrose, and one or more non-native glucosyltransferases herein. Such a reaction composition produces, at least, alpha-glucan comprising 1,3-linkages as disclosed.

The temperature of a reaction composition herein can be controlled, if desired, and can be about 5-50 °C, 20-40 °C, 30-40 °C, 20-30 °C, 20-25 °C, 20 °C, 25 °C, 30 °C, 35 °C, or 40 °C, for example.

The initial concentration of sucrose in a reaction composition herein can be about 20-400 g/L, 75-175 g/L, or 50-150 g/L, for example. In some aspects, the initial sucrose concentration is at least about 50, 75, 100, 150 or 200 g/L, or is about 50-600 g/L, 100-500 g/L, 50-100 g/L, 100-200 g/L, 150-450 g/L, 200-450 g/L, or 250-600 g/L. "Initial concentration of sucrose" refers to the sucrose concentration in a reaction composition just after all the reaction components have been added/combined (e.g., at least water, sucrose, non-native glucosyltransferase enzyme).

The pH of a reaction composition in certain embodiments can be about 4.0-9.0, 4.0-8.5, 4.0-8.0, 5.0-8.0, 5.5-7.5, or 5.5-6.5. In some aspects, the pH can be about 4.0, 4.5, 5.0, 5.5, 6.0, 6.5, 7.0, 7.5, or 8.0. The pH can be adjusted or controlled by the addition or incorporation of a suitable buffer, including but not limited to: phosphate, tris, citrate, or a combination thereof. The buffer concentration in a reaction composition herein can be about 0.1-300 mM, 0.1-100 mM, 10-100 mM, 10 mM, 20 mM, or 50 mM, for example.

A reaction composition can be contained within any vessel (e.g., an inert vessel/container) suitable for applying one or more of the reaction conditions disclosed herein. An inert vessel in some aspects can be of stainless steel, plastic, or glass (or comprise two or more of these components) and be of a size suitable to contain a particular reaction. For example, the volume/capacity of an inert vessel (and/or the volume of a reaction composition herein), can be about, or at least about, 1, 10, 50, 100, 500, 1000, 2500, 5000, 10000, 12500, 15000, or 20000 liters. An inert vessel can optionally be equipped with a stirring device.

A reaction composition herein can contain one, two, or more glucosyltransferase enzymes, for example, just as long that at least one of the enzymes is a non-native glucosyltransferase as presently disclosed. In some embodiments, only one or two glucosyltransferase enzymes is/are comprised in a reaction composition. A glucosyltransferase reaction herein can be, and typically is, cell-free (e.g., no whole cells present).

Any of the features disclosed herein (e.g., above and in the below Examples) regarding a reaction composition can characterize appropriate aspects of a glucan production method herein, and *vice versa.*

The present disclosure also concerns a method for producing alpha-glucan, the method comprising: (a) contacting at least water, sucrose, and at least one non-native glucosyltransferase as disclosed herein that produces an alpha-glucan, whereby alpha-glucan is produced; and b) optionally, isolating the alpha-glucan produced in step (a). Conducting such a method, which can optionally be characterized as a glucan synthesis method, is typically also performed when conducting a reaction composition herein.

A glucan synthesis method as presently disclosed comprises contacting at least water, sucrose, and a non-native glucosyltransferase herein that produces an alpha-glucan. These and optionally other reagents can be added altogether or in any order as discussed below. This step can optionally be characterized as providing a reaction composition comprising water, sucrose and a non-native glucosyltransferase enzyme that synthesizes alpha-glucan. The contacting step herein can be performed in any number of ways. For example, the desired amount of sucrose can first be dissolved in water (optionally, other components may also be added at this stage of preparation, such as buffer components), followed by addition of glucosyltransferase enzyme. The solution may be kept still, or agitated via stirring or orbital shaking, for example. A glucan synthesis method can be performed by batch, fed-batch, continuous mode, or by any variation of these modes.

Completion of a reaction in certain embodiments can be determined visually (e.g., no more accumulation of insoluble glucan), and/or by measuring the amount of sucrose left in the solution (residual sucrose), where a percent sucrose consumption of at least about 90%, 95%, or 99% can indicate reaction completion. A reaction of the disclosed process can be conducted for about 1 hour to about 2, 4, 6, 8, 10, 12, 14, 16, 18, 20, 22, 24, 36, 48, 60, 72, 96, 120, 144, or 168 hours, for example.

The yield of an alpha-glucan produced in some aspects of a glucan synthesis method herein can be at least about 40%, 45%, 50%, 55%, 60%, 65%, 70%, 75%, 80%, 85%, 90%, 91%, 92%, 93%, 94%, 95%, or 96%. Yield in some aspects can be measured based on the glucosyl component of the reaction. In some additional or alternative embodiments, the yield of leucrose can be less than about 18%, 17%, 16%, 15%, 14%, 13%, 12%, 11%, 10%, 9%, 8%, 7%, 6%, 5%, 4%, 3%, 2%, or 1%. Such a yield in alpha-glucan and/or leucrose in some aspects is achieved in a reaction conducted for about 16-24 hours (e.g., ~20 hours), and/or is as measured using HPLC or NIR spectroscopy.

Insoluble alpha-glucan produced in a method herein can optionally be isolated. In certain embodiments, isolating insoluble alpha-glucan can include at least conducting a step of centrifugation and/or filtration. Isolation can optionally further comprise washing alpha-glucan one, two, or more times with water or other aqueous liquid, and/or drying the alpha-glucan product.

An isolated alpha-glucan product herein, as provided in a dry form, can comprise no more than 2.0, 1.5, 1.0, 0.5, 0.25, 0.10, 0.05, or 0.01 wt% water, for example. In some aspects, an alpha-glucan product is provided in an amount of at least 1 gram (e.g., at least about 2.5, 5, 10, 25, 50, 100, 250, 500, 750, 1000, 2500, 5000, 7500, 10000, 25000, 50000, or 100000 g); such an amount can be a dry amount, for example.

A glucan synthesis method in some aspects can further comprise contacting a soluble fraction of the glucosyltransferase reaction, and/or the glucosyltransferase reaction itself, with an alpha-glucosidase enzyme to hydrolyze at least one glycosidic linkage of one or more oligosaccharides present in the soluble fraction and/or glucosyltransferase reaction, thereby increasing the monosaccharide content in the soluble fraction. A soluble fraction herein can be contacted with an alpha-glucosidase after its separation from an insoluble fraction comprising alpha-1,3-glucan, or before its separation (e.g., while it is being formed in the reaction, and/or after completion of the reaction) (i.e., in contacting step [a] and/or after separation step [b]). A soluble fraction can be a filtrate or supernatant, for example, of a glucosyltransferase reaction, and is typically obtained following the completion of insoluble alpha-1,3-glucan synthesis. Examples of suitable alpha-glucosidases herein include those comprising an amino acid sequence that (i) is 100% identical to, or at least about 90%, 91%, 92%, 93%, 94%, 95%, 96%, 97%, 98%, or 99% identical to, any of SEQ ID NOs:68-81, and (ii) has hydrolytic activity toward alpha-1,5 glucosyl-fructose linkages, alpha-1,3 glucosyl-glucose linkages, and/or alpha-1,6 glucosyl-glucose linkages in saccharides.

Any of the disclosed conditions for synthesizing an alpha-glucan, such as the foregoing or those described in the below Examples, can be applied to practicing a reaction composition as presently disclosed (and *vice versa*)*,* and/or used to characterize features/activity of a non-native glucosyltransferase, accordingly.

A method of preparing a polynucleotide sequence encoding a non-native glucosyltransferase herein may comprise:
(a) identifying a polynucleotide sequence encoding a parent glucosyltransferase that (i) comprises an amino acid sequence that is at least about 90% identical to positions 55-960 of SEQ ID NO:4, and (ii) synthesizes alpha-glucan comprising 1,3-linkages; and
(b) modifying the polynucleotide sequence identified in step (a) to substitute at least two or three amino acids of the parent glucosyltransferase at positions corresponding with amino acid residues Gln-588, Phe-607 and/or Arg-741 of SEQ ID NO:62, wherein:
   (i) the amino acid substitution at the position corresponding with amino acid residue Gln-588 is with a Leu, Ala, or Val residue;
   (ii) the amino acid substitution at the position corresponding with amino acid residue Phe-607 is with a Trp or Asn residue; and/or
   (iii) the amino acid substitution at the position corresponding with amino acid residue Arg-741 is with a Ser or Thr residue, thereby providing a polynucleotide sequence encoding a non-native glucosyltransferase that has:
      (i) an alpha-glucan yield that is higher than the alpha-glucan yield of the parent glucosyltransferase, and/or
      (ii) a leucrose yield that is lower than the leucrose yield of the parent glucosyltransferase.
Further a polynucleotide prepared in this manner may be used in a method of expressing the non-native glucosyltransferase encoded by the polynucleotide. Such an expression method can follow any heterologous protein expression method as known in the art, for example. Such a method may be seen as a method of increasing the product yield of a glucosyltransferase.

Identification step (a) herein can, in some instances, comprise identifying an amino acid sequence of a parent glucosyltransferase enzyme. A polynucleotide sequence could be determined from this amino acid sequence according to the genetic code (codons), such as the genetic code used in the species from which the parent glucosyltransferase was identified.

Identifying a polynucleotide encoding a parent glucosyltransferase can be performed (a) *in silico,* (b) with a method comprising a nucleic acid hybridization step, (c) with a method comprising a protein sequencing step, and/or (d) with a method comprising a protein binding step, for example.

Regarding *in silico* detection, the amino acid sequences of candidate parent glucosyltransferase enzymes (and/or nucleotide sequences encoding such glucosyltransferase enzymes) stored in a computer or database (e.g., public databases such as GENBANK, EMBL, REFSEQ, GENEPEPT, SWISS-PROT, PIR, PDB) can be reviewed *in silico* to identify a glucosyltransferase enzyme comprising an amino acid sequence with a percent sequence identity as described above for a parent glucosyltransferase. Such review could comprise using any means known in the art such as through use of an alignment algorithm or software as described above (e.g., BLASTN, BLASTP, ClustalW, ClustalV, Clustal-Omega, EMBOSS).

Identifying a parent glucosyltransferase can be performed via a method comprising a nucleic acid hybridization step. Such a method can comprise using DNA hybridization (e.g., Southern blot, dot blot), RNA hybridization (e.g., northern blot), or any other method that has a nucleic acid hybridization step (e.g., DNA sequencing, PCR, RT-PCR, all of which may comprise hybridization of an oligonucleotide), for example. A polynucleotide sequence encoding SEQ ID NO:4 or a subsequence thereof (e.g., positions 55-960 of SEQ ID NO:4) can be used as a probe, for example, in such a hybridization. Conditions and parameters for carrying out hybridization methods in general are well known and disclosed, for example, in Sambrook J, Fritsch EF and Maniatis T, Molecular Cloning: A Laboratory Manual, Cold Spring Harbor Laboratory: Cold Spring Harbor, NY (1989); Silhavy TJ, Bennan ML and Enquist LW, Experiments with Gene Fusions, Cold Spring Harbor Laboratory: Cold Spring Harbor, NY (1984); Ausubel FM et al., Current Protocols in Molecular Biology, published by Greene Publishing Assoc. and Wiley-Interscience, Hoboken, NJ (1987); and Innis MA, Gelfand DH, Sninsky JJ and White TJ (Editors), PCR Protocols: A Guide to Methods and Applications, Academic Press, Inc., San Diego, CA (1990).

Identifying a parent glucosyltransferase can be performed via a method comprising a protein sequencing step. Such a protein sequencing step can comprise one or more procedures such as N-terminal amino acid analysis, C-terminal amino acid analysis, Edman degradation, or mass spectrometry, for example.

Identifying a parent glucosyltransferase can be performed via a method comprising a protein binding step. Such a protein binding step can be performed using an antibody that binds to a motif or epitope within SEQ ID NO:4 (e.g., within positions 55-960 of SEQ ID NO:4), for example.

A suitable modification of a polynucleotide in step (b) can be made following any DNA manipulation technique known in the art. Modifying step (b) can optionally be performed *in silico,* followed by synthesis of the polynucleotide sequence encoding a non-native glucosyltransferase. For example, a polynucleotide sequence identified in step (a) can be manipulated *in silico* using a suitable sequence manipulation program/software (e.g., VECTOR NTI, Life Technologies, Carlsbad, CA; DNAStrider; DNASTAR, Madison, WI). Following such virtual manipulation, the modified polynucleotide sequence can be artificially synthesized by any suitable technique (e.g., annealing-based connection of oligonucleotides, or any technique disclosed in Hughes et al., Methods Enzymol. 498:277-309). It should be appreciated that the foregoing methodology is not believed to necessarily rely on having a pre-existing polynucleotide (encoding a parent glucosyltransferase) in hand.

Modifying step (b) can optionally be performed using a physical copy of a polynucleotide sequence identified in step (a) encoding a parent glucosyltransferase. As an example, such a polynucleotide can serve as a template for amplification using primers designed in a manner such that the amplified product encodes a non-native glucosyltransferase herein (e.g., refer to Innis et al., *ibid*.).

### EXAMPLES

The present disclosure is further exemplified in the following Examples. It should be understood that these Examples, while indicating certain preferred aspects herein, are given by way of illustration only. From the above discussion and these Examples, one skilled in the art can ascertain the essential characteristics of the disclosed embodiments and can make various changes and modifications to adapt the disclosed embodiments to various uses and conditions.

### EXAMPLE 1

### Analysis of Amino Acid Sites Affecting Glucosyltransferase Selectivity toward Alpha-Glucan Synthesis

This Example describes screening for glucosyltransferase variants with improved selectivity toward alpha-glucan synthesis from sucrose. Another aim of this screening was to identify glucosyltransferase variants that exhibit reduced synthesis of by-products such as leucrose and gluco-oligosaccharides. Variants having either or both of these yield properties were identified.

The amino acid sequence of the glucosyltransferase used to prepare amino acid substitutions in this Example was SEQ ID NO:4 (GTF 6855), which essentially is an N-terminally truncated (signal peptide and variable region removed) version of the full-length wild type glucosyltransferase (represented by SEQ ID NO:62) from *Streptococcus salivarius* SK126 (see Table 1). Substitutions made in SEQ ID NO:4 can be characterized as substituting for native amino acid residues, as each amino acid residue/position of SEQ ID NO:4 (apart from the Met-1 residue of SEQ ID NO:4) corresponds accordingly with an amino acid residue/position within SEQ ID NO:62. In reactions comprising at least sucrose and water, the glucosyltransferase of SEQ ID NO:4 typically produces alpha-glucan having about 100% alpha-1,3 linkages and a DP_{w} of 400 or greater (e.g., refer to U.S. Patent Nos. 8871474 and 9169506, and U.S. Pat. Appl. Publ. No. 2017/0002336). This alpha-glucan product, which is insoluble, can be isolated following enzymatic synthesis via filtration, for example.

To summarize this Example, GTF 6855 variants (each with a single amino acid substitution) from site evaluation libraries (SEL) were each bacterially expressed, purified, and normalized to a concentration of 100 ppm. Each enzyme preparation was then screened (in triplicate) using sucrose as substrate in alpha-1,3 glucan synthesis reactions. In addition to determining the amount of alpha-1,3 glucan polymer produced in each reaction, the soluble sugar products (fructose, glucose, leucrose, gluco-oligosaccharides) and residual sucrose of each reaction were analyzed by HPLC after about a 20-hour incubation.

Plasmids for individually expressing various single amino acid-substituted variants of GTF 6855 (SEQ ID NO:4) in a *Bacillus subtilis* host were prepared. Such plasmids were prepared as follows. A DNA expression cassette having (operably linked in 5'-to-3' order) the B. *subtilis* aprE promoter, a codon-optimized sequence encoding SEQ ID NO:4 (GTF 6855), and a BPN' terminator was synthesized. This expression cassette was cloned into the pHYT replicating shuttle vector (forming pHYT-GTF6855) and transformed into *B*. *subtilis* CBS12-1. The pHYT vector was derived from pHY300PLK (Takara) by adding a terminator sequence (SEQ ID NO:67) after the tetracycline resistance gene using the *BstEII* and *EcoRI* sites. The *HindIII* site in pHY300PLK had been removed by cloning a linker sequence (not shown) into the *BamHI* and *HindIII* sites. The pHYT-GTF6855 plasmid was amplified and used for generating SELs. The resulting plasmids encoding single-amino acid substituted GTFs were sequenced to verify each substitution.

To produce GTF 6855 (SEQ ID NO:4) and single amino acid-substituted variants thereof, *B. subtilis* individually transformed with pHYT-GTF6855 or mutated versions thereof were cultivated in Tryptone Soya Broth (Oxoid Ltd., UK) and Grant's II medium. Heart infusion agar plates (Difco Laboratories, MI) were used to select transformants. Plasmid integrity was maintained by the addition of 25 µg/mL tetracycline. Each GTF targeted for expression was detected in the growth medium after incubation for about 6 hours at 37 °C. After centrifugation and filtration, culture supernatants with expressed GTF were obtained. GTF enzyme present in the supernatant was purified to apparent homogeneity by affinity chromatography using washed (2x MILLIQ 1 x 25 mM NaH₂PO₄ pH 5.7 with intermediate centrifugation steps 100 x g) SUPERDEX 200 resin (GE Healthcare). Each GTF was eluted with a 15% solution of Dextran T1 (Pharmacosmos) in 25 mM NaH₂PO₄ pH 5.7 by centrifugation 100 x g. Each purified GTF was dialyzed against 25 mM NaH₂PO₄ pH 5.7 buffer (at least 100x) using a Harvard Apparatus 96-well DISPODIALYZER (10000-Dalton MWCO).

After dialysis, GTF enzyme concentration was determined by OD280 using purified GTF 6855 as a standard. Normalization of each purified GTF to 100 ppm was achieved by diluting appropriately with 25 mM NaH₂PO₄ pH 5.7. Protein concentration for each sample was confirmed using an AGILENT 1200 (Agilent Technologies) HPLC equipped with an AGILENT BIO SEC3 guard-column column (3 µm 100Å (4.6x50 mm). Five (5) µL of sample was injected onto the column for each determination. Compounds were eluted with isocratic flow of 25 mM KH₂PO₄ pH 6.8 + 0.1 M NaCl for 1.3 min at 0.5 mL/min flow rate.

Each GTF (GTF 6855 and each variant thereof) was entered into a reaction with sucrose to determine yield and selectivity. Each reaction was performed as follows: 37.5 µL of 100 ppm enzyme sample (ppm based on a BSA calibration curve) was added to 262.5 µL of 86 g/L sucrose (75 g/L final) in 20 mM Na₂HPO₄/NaH₂PO₄ pH 5.7 and incubated overnight (about 20 hours) at 30 °C. After this incubation, each reaction was quenched by incubation for 1 hour at 80 °C. A 200-µL aliquot of each quenched reaction was filtered *in vacuo* via a 0.45-µm filter plate (Millipore 0.45-µm Hydrophilic) and each filtrate was diluted 5x (10 µL sample + 40 µL 20 mM Na₂HPO₄/NaH₂PO₄) in preparation for HPLC sugar analysis.

Sucrose, glucose, fructose, leucrose and relative oligosaccharide concentrations in each diluted filtrate were determined using an AGILENT 1200 (Agilent Technologies) HPLC equipped with a 150 x 7.80 mm PHENOMENEX REZEX RNM carbohydrate Na⁺ 8% column PHENOMENX KRUDKATCHER 0.5-µm guard column. The column was operated at 80 °C with an isocratic flow-rate of 0.9 mL/min with 10 mM Na₂HPO₄ /NaH₂PO₄ pH 6.7 (5 min per sample). Five µL of diluted sample was injected. Appropriate sucrose, glucose, fructose, and leucrose calibration curves were used to determine sugar concentrations. A mixture of purified gluco-oligosaccharides was used to determine oligomer concentration.

The profiles of reactions (~20 hours) as measured via the above methodology are provided in Table 3.

**Table 3. Product Profiles of GTF 6855 (SEQ ID NO:4) and Single Amino Acid-Substituted Variants thereof**

| Plate 1^{a} | | | | | | | |
|---|---|---|---|---|---|---|---|
| GTF | Sucrose (g/L)^{d} | Leucrose (g/L)^{d} | Glucose (g/L)^{d} | Fructose (g/L)^{d} | Oligomers (g/L)^{d,e} | Alpha-1,3 Glucan^{f} Yieldⁱ | Fructose Balance |
| 6855^{b} | 1.6 | 21.1 | 6.3 | 28.9 | 9.1 | 31% | 97% |
| 6855^{b} | 1.6 | 21.3 | 6.3 | 29.1 | 10.5 | 27% | 98% |
| 6855^{b} | 1.6 | 21.2 | 6.3 | 29.3 | 10.0 | 29% | 98% |
| 6855^{b} | 1.6 | 21.1 | 6.3 | 28.9 | 10.8 | 27% | 97% |
| V186A^{c} | 1.6 | 21.3 | 6.4 | 28.8 | 10.7 | 27% | 97% |
| V186M | 1.6 | 21.4 | 6.4 | 28.7 | 10.6 | 27% | 97% |
| E194C | 1.6 | 21.2 | 6.3 | 29.0 | 9.4 | 30% | 98% |
| L434N | 1.9 | 22.7 | 7.1 | 28.4 | 12.7 | 18% | 99% |
| A472C | 31.0 | 2.6 | 2.5 | 23.8 | 4.6 | 38% | 99% |
| A472S | 5.3 | 2.8 | 13.9 | 36.5 | 9.1 | 31% | 97% |
| A510E | 8.5 | 5.4 | 5.5 | 34.5 | 5.6 | 53% | 100% |
| A510E | 1.9 | 6.5 | 5.6 | 36.7 | 6.1 | 58% | 98% |
| A510I | 4.3 | 6.8 | 5.4 | 35.2 | 5.4 | 57% | 98% |
| A510V | 1.7 | 9.5 | 6.4 | 35.6 | 6.8 | 51% | 99% |
| L513Y | 1.4 | 10.3 | 4.2 | 35.3 | 7.2 | 54% | 99% |
| M529L | 1.9 | 10.4 | 4.2 | 35.2 | 10.9 | 44% | 99% |
| K578M | 1.6 | 21.0 | 6.4 | 28.8 | 10.8 | 27% | 97% |
| Y605W | 6.1 | 8.0 | 2.6 | 33.3 | 5.4 | 59% | 97% |
| F607N | 8.4 | 11.4 | 4.1 | 30.5 | 7.1 | 45% | 98% |
| F607W | 9.1 | 4.6 | 3.8 | 33.9 | 8.6 | 49% | 98% |
| N613I | 4.5 | 7.7 | 6.4 | 35.8 | 14.8 | 29% | 101% |
| N613M | 2.7 | 11.0 | 5.3 | 34.6 | 12.1 | 37% | 100% |
| N613T | 1.7 | 10.3 | 4.6 | 35.0 | 7.1 | 53% | 98% |
| N613V | 2.8 | 0.0 | 6.3 | 37.3 | 12.1 | 48% | 92% |
| Q616E | 3.9 | 2.4 | 5.8 | 37.3 | 8.8 | 53% | 97% |
| K625A | 1.5 | 21.2 | 6.3 | 29.4 | 9.9 | 29% | 99% |
| K625M | 1.5 | 21.3 | 6.3 | 29.3 | 10.6 | 27% | 99% |
| S631T | 5.4 | 11.4 | 4.6 | 32.0 | 7.6 | 46% | 97% |
| T635H | 4.1 | 11.0 | 5.0 | 32.7 | 8.2 | 46% | 97% |
| T635W | 13.1 | 8.5 | 4.5 | 29.6 | 7.0 | 42% | 98% |
| I636H | 7.0 | 11.7 | 5.0 | 31.1 | 8.1 | 42% | 98% |
| D947G | 2.4 | 19.1 | 6.1 | 29.8 | 9.9 | 31% | 98% |
| F951Y | 4.0 | 1.5 | 9.9 | 38.0 | 15.4 | 28% | 97% |
| E849M | 1.4 | 20.7 | 6.2 | 29.5 | 10.4 | 29% | 98% |
| Q1007A | 1.4 | 19.4 | 6.2 | 30.2 | 10.1 | 31% | 98% |
| D1003G | 13.8 | 10.7 | 4.6 | 28.3 | 5.4 | 42% | 98% |
| A1022M | 1.7 | 20.6 | 6.2 | 29.3 | 12.2 | 24% | 98% |
| D1028L | 1.6 | 22.1 | 6.6 | 28.9 | 11.6 | 23% | 99% |
| D1028Q | 1.6 | 21.7 | 6.5 | 29.4 | 10.9 | 26% | 99% |
| A1057H | 1.5 | 21.4 | 6.4 | 29.2 | 10.6 | 27% | 98% |
| N1096A | 1.6 | 22.4 | 6.6 | 28.6 | 10.7 | 25% | 98% |
| E1132A | 1.5 | 21.4 | 6.4 | 29.2 | 10.6 | 27% | 98% |
| E1132H | 1.5 | 21.3 | 6.4 | 29.2 | 10.5 | 27% | 98% |
| E1132K | 1.5 | 21.4 | 6.4 | 29.2 | 10.4 | 27% | 98% |
| E1132R | 1.5 | 21.6 | 6.4 | 29.1 | 10.8 | 26% | 99% |
| L1212N | 1.5 | 20.9 | 6.3 | 29.5 | 10.4 | 28% | 98% |
| T1431M | 1.5 | 21.4 | 6.3 | 29.4 | 10.5 | 27% | 99% |
| A1442R | 1.5 | 21.3 | 6.4 | 29.1 | 10.6 | 27% | 98% |
| Dead^{g} | 79.4 | 0.0 | 0.0 | 0.0 | 0.0 | 0% | 100% |
| Blank^{h} | 79.7 | 0.0 | 0.1 | 0.0 | 0.0 | 0% | 100% |
| Blank^{h} | 80.1 | 0.0 | 0.0 | 0.0 | 0.0 | 0% | 100% |
| | | | | | | | |

| Plate 2^{a} | | | | | | | |
|---|---|---|---|---|---|---|---|
| GTF | Sucrose (g/L)^{d} | Leucrose (g/L)^{d} | Glucose (g/L)^{d} | Fructose (g/L)^{d} | Oligomers (g/L)^{d,e} | Alpha-1,3 Glucan^{f} Yieldⁱ | Fructose Balance |
| 6855^{b} | 1.4 | 20.1 | 6.4 | 28.2 | 10.0 | 29% | 99% |
| 6855^{b} | 1.4 | 20.1 | 6.4 | 28.2 | 10.1 | 28% | 99% |
| 6855^{b} | 1.4 | 20.0 | 6.3 | 28.3 | 10.3 | 28% | 99% |
| 6855^{b} | 1.5 | 20.2 | 6.3 | 28.2 | 10.0 | 29% | 100% |
| Y219C^{c} | 1.5 | 20.6 | 6.5 | 27.7 | 10.7 | 25% | 99% |
| E243H | 1.4 | 20.3 | 6.3 | 28.2 | 10.1 | 28% | 100% |
| L373A | 2.4 | 11.3 | 11.2 | 27.4 | 21.6 | -7% | 87% |
| L373Q | 4.0 | 7.5 | 10.7 | 28.4 | 21.5 | -2% | 87% |
| L373V | 2.5 | 11.6 | 11.5 | 27.5 | 21.8 | -9% | 88% |
| A377I | 2.9 | 15.5 | 6.6 | 29.3 | 11.3 | 29% | 98% |
| D425Q | 1.8 | 15.3 | 5.3 | 30.3 | 9.6 | 39% | 99% |
| L428V | 5.3 | 10.5 | 6.2 | 30.8 | 8.2 | 42% | 98% |
| N475F | 6.1 | 26.8 | 20.5 | 24.9 | 7.2 | -16% | 106% |
| N475W | 1.5 | 61.8 | 7.5 | 9.1 | 1.9 | -8% | 106% |
| L513F | 1.0 | 10.9 | 4.6 | 33.3 | 7.1 | 55% | 99% |
| L513W | 1.3 | 11.5 | 4.9 | 32.4 | 8.9 | 48% | 98% |
| M529N | 3.5 | 11.6 | 4.8 | 31.6 | 7.6 | 49% | 99% |
| I608Y | 2.4 | 15.7 | 5.7 | 29.9 | 9.8 | 35% | 99% |
| N613G | 2.2 | 10.5 | 5.0 | 33.5 | 10.6 | 43% | 101% |
| N613L | 2.9 | 13.3 | 5.0 | 32.1 | 11.7 | 35% | 102% |
| D617E | 8.4 | 10.2 | 6.9 | 29.8 | 9.0 | 34% | 99% |
| E621T | 1.5 | 18.6 | 6.0 | 29.1 | 10.4 | 30% | 100% |
| I623H | 69.8 | 0.2 | 1.4 | 3.3 | 0.0 | 4% | 101% |
| I627W | 7.7 | 12.2 | 5.2 | 28.9 | 7.9 | 40% | 99% |
| S631D | 9.8 | 12.3 | 5.7 | 27.5 | 8.0 | 35% | 98% |
| S631E | 10.1 | 12.6 | 5.6 | 27.3 | 8.0 | 35% | 99% |
| S631R | 6.7 | 12.3 | 5.4 | 28.7 | 8.1 | 40% | 97% |
| G633W | 7.0 | 7.2 | 5.5 | 31.9 | 8.5 | 46% | 99% |
| F634A | 7.4 | 8.4 | 5.7 | 30.8 | 8.2 | 43% | 98% |
| T635E | 1.6 | 17.2 | 6.0 | 29.9 | 9.5 | 35% | 100% |
| T635I | 1.5 | 17.4 | 6.2 | 30.5 | 10.1 | 32% | 102% |
| T635Y | 13.8 | 8.0 | 4.6 | 28.0 | 6.7 | 43% | 99% |
| A510E | 2.5 | 5.9 | 5.5 | 34.8 | 4.3 | 66% | 99% |
| N904E | 5.7 | 6.9 | 12.6 | 32.5 | 13.5 | 15% | 98% |
| K930G | 1.4 | 19.8 | 6.2 | 28.4 | 10.0 | 30% | 99% |
| K930V | 1.4 | 19.6 | 6.3 | 28.6 | 10.0 | 30% | 100% |
| D947F | 1.4 | 20.3 | 6.2 | 27.8 | 9.9 | 29% | 99% |
| D947I | 1.4 | 19.9 | 6.3 | 28.6 | 10.7 | 27% | 100% |
| D947K | 1.4 | 19.9 | 6.2 | 28.6 | 9.7 | 30% | 100% |
| D947N | 1.4 | 20.5 | 6.3 | 27.9 | 10.0 | 28% | 99% |
| D947Q | 1.4 | 19.5 | 6.2 | 28.4 | 9.6 | 31% | 99% |
| D947S | 1.3 | 18.9 | 6.1 | 28.8 | 9.4 | 33% | 99% |
| D947V | 1.4 | 19.8 | 6.2 | 28.3 | 9.7 | 30% | 99% |
| D947Y | 1.4 | 20.7 | 6.3 | 28.1 | 10.0 | 28% | 100% |
| Q1007S | 1.3 | 18.3 | 6.1 | 29.1 | 9.6 | 33% | 99% |
| D1003N | 3.6 | 13.1 | 5.7 | 30.5 | 9.8 | 38% | 99% |
| I1026H | 1.4 | 19.4 | 6.2 | 28.7 | 9.7 | 31% | 100% |
| D1028A | 1.5 | 20.1 | 6.5 | 28.4 | 10.8 | 26% | 100% |
| D1028M | 1.5 | 20.4 | 6.6 | 28.1 | 11.1 | 24% | 100% |
| V1037A | 1.5 | 20.2 | 6.4 | 28.4 | 10.3 | 28% | 100% |
| K1041A | 4.3 | 19.6 | 6.5 | 27.0 | 10.7 | 23% | 99% |
| K1041M | 1.5 | 20.5 | 6.4 | 28.0 | 10.5 | 26% | 100% |
| D1080M | 1.4 | 20.0 | 6.4 | 28.3 | 10.1 | 29% | 99% |
| F1244P | 1.4 | 19.6 | 6.3 | 28.6 | 9.9 | 30% | 100% |
| F1244Q | 1.4 | 19.7 | 6.4 | 28.6 | 9.9 | 30% | 100% |
| T1431Q | 1.4 | 20.0 | 6.2 | 28.5 | 8.9 | 33% | 100% |
| G1484P | 1.5 | 20.1 | 6.3 | 28.5 | 9.2 | 31% | 100% |
| W1437N | 1.4 | 19.5 | 6.0 | 28.9 | 8.4 | 35% | 100% |
| Dead^{g} | 75.5 | 0.0 | 0.0 | 0.0 | 0.0 | 0% | 100% |
| Blank^{h} | 75.5 | 0.0 | 0.0 | 0.0 | 0.0 | 0% | 100% |
| Blank^{h} | 76.0 | 0.0 | 0.0 | 0.0 | 0.5 | -2% | 100% |

| | | | | | | | |
|---|---|---|---|---|---|---|---|
| ^{a} Glucan synthesis reactions were run in microtiter plate format (two plates). ^{b} GTF 6855, SEQ ID NO:4. Reactions with this GTF were run in quadruplicate per plate. ^{c} Each listed GTF with a substitution is a version of GTF 6855 comprising a substitution at a respective position, where the position number is in correspondence with the residue numbering of SEQ ID NO:62. The wild type residue is listed first (before residue position number) and the substituting residue is listed second (after the residue position number) (this "wild type residue-position number-variant residue" annotation format applies throughout the present disclosure). ^{d} Sucrose, leucrose, glucose, fructose and oligomers were measured as present in filtrate prepared post reaction. ^{e} "Oligomers", gluco-oligosaccharides (believed to all or mostly be of DP ≤ 7 or 8). ^{f} Insoluble alpha-1,3 glucan product. ^{g} GTF with destroyed activity was entered into the reaction. ^{h} No GTF was added to the reaction. ⁱ Alpha-glucan yield based on glucosyl. | | | | | | | |

Based on the data in Table 3, it is apparent that certain single amino acid substitutions in GTF 6855 (SEQ ID NO:4) can increase this enzyme's yield of alpha-1,3-glucan and/or decrease its leucrose yield in glucan synthesis reactions, for example.

### EXAMPLE 2

### Analysis of the Effects of Single Amino Acid Substitutions on Other Glucosyltransferases

This Example describes the effects of certain single amino acid substitutions on the activities of glucosyltransferases other than GTF 6855 (SEQ ID NO:4). In general, it appears that substitutions corresponding to (or similar to) those observed in Example 1 having a significant effect on alpha-glucan and/or leucrose yields may be useful for imparting similar effects to different glucosyltransferases.

### Phe-607-Tyr

Example 1 demonstrated, for example, that substitutions in GTF 6855 (SEQ ID NO:4) at the position corresponding to position 607 of SEQ ID NO:62 affected enzyme activity (Table 3). In particular, substitutions of the Phe residue with an Asn or Trp residue both had significant effects on alpha-1,3 glucan yield (increased) and leucrose yield (decreased) compared to the respective yields of the non-substituted enzyme.

To test whether a similar substitution could similarly affect yields in a different GTF, a substitution was made at a position in GTF 7527 (GTFJ, SEQ ID NO:65) corresponding to position 607 of SEQ ID NO:62, exchanging a Phe for a Tyr residue. GTF 7527 (SEQ ID NO:65) essentially is an N-terminally truncated (signal peptide and variable region removed) version of the full-length wild type glucosyltransferase (represented by SEQ ID NO:60) from *Streptococcus salivarius* (see Table 1). Substitutions made in SEQ ID NO:65 can be characterized as substituting for native amino acid residues, as each amino acid residue/position of SEQ ID NO:65 (apart from the Met-1 residue of SEQ ID NO:65) corresponds accordingly with an amino acid residue/position within SEQ ID NO:60. In reactions comprising at least sucrose and water, the glucosyltransferase of SEQ ID NO:65 typically produces alpha-glucan having about 100% alpha-1,3 linkages and a DPw of 400 or greater (e.g., refer to U.S. Patent Nos. 8871474 and 9169506, and U.S. Pat. Appl. Publ. No. 2017/0002336). Glucan synthesis reactions were prepared as follows using GTF 7527 (SEQ ID NO:65) or a version thereof comprising a Phe-to-Tyr substitution at the position corresponding to position 607 of SEQ ID NO:62: vessel, 250-mL indented shake flask agitated at 100 rpm; initial pH, 5.5; reaction volume, 50 mL; sucrose, 100.1 g/L; GTF, 100 U/L; KH₂PO4, 25 mM; temperature, 25 °C; time, 20 hours. The profiles of each reaction (as measured via methodology similar to that disclosed in Example 1), which were run in duplicate, are provided in Table 4.

**Table 4. Product Profiles of GTF 7527 (SEQ ID NO:65) and a Single Amino Acid-Substituted Variant thereof**

| GTF | Sucrose Conv. | Yield Alpha-Glucan^{d} based on Glucosyl | Leucrose Yield | Glucose Yield | Oligomer^{c} Yield | Fructose balance |
|---|---|---|---|---|---|---|
| 7527^{a} | 99.7% | 29.24% | 42% | 4.20% | 28% | 105.62% |
| 7527 | 99.8% | 22.21% | 43% | 6.26% | 29% | 109.02% |
| F607Y^{b} | 99.8% | 64.92% | 16% | 3.33% | 15% | 102.73% |
| F607Y | 99.8% | 62.97% | 17% | 3.35% | 17% | 109.17% |

| | | | | | | |
|---|---|---|---|---|---|---|
| ^{a} GTF 7527, SEQ ID NO:65. ^{b} F607Y, version of GTF 7527 (SEQ ID NO:65) comprising a Phe-to-Tyr substitution at the position corresponding to position 607 of SEQ ID NO:62. ^{c} "Oligomer", gluco-oligosaccharides (believed to all or mostly be of DP ≤ 7 or 8). ^{d} "Alpha-Glucan", insoluble alpha-1,3 glucan. | | | | | | |

Based on the data in Table 4, it is apparent that the F607Y substitution in GTF 7527 (SEQ ID NO:65) can increase this enzyme's yield of alpha-1,3-glucan and/or decrease its leucrose yield in glucan synthesis reactions, for example.

### Ala-510-Glu, Ala-510-Val, or Ala-510-Cys

Example 1 demonstrated, for example, that substitutions in GTF 6855 (SEQ ID NO:4) at the position corresponding to position 510 of SEQ ID NO:62 affected enzyme activity (Table 3). In particular, substitutions of the Ala residue with a Glu, Ile, or Val residue all had significant effects on alpha-1,3 glucan yield (increased) and leucrose yield (decreased) compared to the respective yields of the non-substituted enzyme.

To test whether these or similar substitutions could similarly affect yields in different GTFs, substitutions were made at positions in GTFs 2919 (SEQ ID NO:28), 0427 (SEQ ID NO:26), 5926 (SEQ ID NO:14), 0847 (SEQ ID NO:2), 0544 (SEQ ID NO:12), 2379 (SEQ ID NO:6), 5618 (SEQ ID NO:18), 4297 (SEQ ID NO:16), 1366 (SEQ ID NO:24), and 6907 (SEQ ID NO:36) corresponding to position 510 of SEQ ID NO:62, exchanging an Ala for a Glu, Val, or Cys residue. Each of these GTFs essentially is an N-terminally truncated (signal peptide and variable region removed) version of a full-length wild type glucosyltransferase (e.g., refer to respective GENBANK annotation information, such as that listed in Table 1). Substitutions made in each of SEQ ID NOs:28, 26, 14, 2, 12, 6, 18, 16, 24 and 36 can be characterized as substituting for native amino acid residues, as each amino acid residue/position of these sequences (apart from the Met-1 residues of each) corresponds accordingly with an amino acid residue/position within each respective full-length wild type glucosyltransferase counterpart. Table 2 lists the alpha-glucan typically produced by each of SEQ ID NOs:28, 26, 14, 2, 12, 6, 18, 16, 24 and 36 in reactions comprising at least sucrose and water.

Preparation of GTF 2919 (SEQ ID NO:28), 0427 (SEQ ID NO:26), 5926 (SEQ ID NO:14), 0847 (SEQ ID NO:2), 0544 (SEQ ID NO:12), 2379 (SEQ ID NO:6), 5618 (SEQ ID NO:18), 4297 (SEQ ID NO:16), 1366 (SEQ ID NO:24), or 6907 (SEQ ID NO:36), or versions thereof comprising a substitution at the position corresponding to position 510 of SEQ ID NO:62 was performed as follows. Codon-optimized (for *E. coli*) sequences encoding each of these GTFs were individually cloned into a suitable plasmid for bacterial expression. Each construct was then transformed into *E. coli* BL21-Al (Invitrogen, Carlsbad, CA). Transformed strains were grown in 10 mL auto-induction medium (10 g/L Tryptone, 5 g/L Yeast Extract, 5 g/L NaCl, 50 mM Na₂HPO₄, 50 mM KH₂PO₄, 25 mM (NH₄)₂SO₄, 3 mM MgSO₄, 0.75% glycerol, 0.075% glucose, 0.05% arabinose) containing 100 mg/L ampicillin at 37 °C for 20 hours under 200 rpm agitation. The cells were harvested by centrifugation at 8000 rpm at 4 °C and resuspended in 1 mL of 20 mM sodium phosphate buffer pH 6.0 with CelLytic^{™} Express (Sigma, St. Louise, MO) according to the manufacturer's instructions. In addition, resuspended cells were subjected to no less than one freeze-thaw cycle to ensure cell lysis. Lysed cells were centrifuged for 10 minutes at 12,000g at room temperature. Each resulting supernatant was analyzed by SDS-PAGE to confirm expression of the particular GTF enzyme being expressed. Each supernatant was kept on ice at 4 °C until enzyme activity could be determined (within 1 hour), and/or stored at -20 °C.

Glucan synthesis reactions were prepared, and the products thereof analyzed, largely according to the disclosure of U.S. Pat. Appl. Publ. No. 2014/0087431. Each reaction was run for 24-30 hours. The profiles of each reaction are provided in Table 5.

**Table 5. Product Profiles of Various GTFs and Single Amino Acid-Substituted Variants thereof**

| GTF | Sucrose Conv. | Yield Alpha-Glucan based on Glucosyl | Leucrose Yield | Glucose Yield | Oligomer Yield | Fructose balance |
|---|---|---|---|---|---|---|
| **2919^{a}** | 92% | 20% | 28% | 15% | 37% | 90% |
| A510E^{b} | 98% | 40% | 13% | 15% | 31% | 93% |
| A510V^{b} | 97% | 45% | 15% | 15% | 26% | 84% |
| A510C^{b} | 95% | 35% | 19% | 15% | 32% | 87% |
| | | | | | | |
| **0427^{a}** | 96% | 15% | 33% | 11% | 41% | 97% |
| A510E^{b} | 96% | 1.0% | 40% | 16% | 43% | 104% |
| A510V^{b} | poor conversion | | | | | |
| A510C^{b} | 96% | 9% | 30% | 12% | 50% | 97% |
| | | | | | | |
| **5926^{a}** | 97% | 12% | 37% | 11% | 41% | 93% |
| A510E^{b} | 96% | 12% | 40% | 14% | 34% | 94% |
| A510V^{b} | 97% | 25% | 31% | 14% | 31% | 81% |
| A510C^{b} | 97% | -1% | 35% | 14% | 52% | 97% |
| | | | | | | |
| **0847^{a}** | 97% | 18% | 33% | 11% | 38% | 92% |
| A510E^{b} | 98% | 11% | 35% | 14% | 40% | 95% |
| A510V^{b} | 80% | 32% | 21% | 16% | 31% | 80% |
| A510C^{b} | 97% | 10% | 33% | 13% | 44% | 97% |
| | | | | | | |
| **0544^{a}** | 99% | 37% | 22% | 8% | 33% | 86% |
| A510E^{b} | 93% | 46% | 21% | 8% | 25% | 85% |
| A510V^{b} | poor conversion | | | | | |
| A510C^{b} | 92% | 39% | 16% | 9% | 37% | 90% |
| | | | | | | |
| **2379^{a}** | 95% | 4% | 30% | 18% | 48% | 92% |
| A510E^{b} | 97% | -2% | 23% | 23% | 56% | 93% |
| A510V^{b} | 94% | 5% | 20% | 23% | 52% | 82% |
| A510C^{b} | 93% | -10% | 37% | 21% | 53% | 101% |
| | | | | | | |
| **5618^{a}** | 99% | 80% | 10% | 5% | 5% | 89% |
| A510E^{b} | 94% | 82% | 5% | 4% | 9% | 93% |
| A510V^{b} | 99% | 83% | 7% | 5% | 5% | 78% |
| A510C^{b} | 98% | 83% | 9% | 4% | 4% | 96% |
| | | | | | | |
| **4297^{a}** | 97% | 78% | 12% | 6% | 4% | 86% |
| A510E^{b} | 99% | 84% | 7% | 4% | 5% | 83% |
| A510V^{b} | 99% | 78% | 8% | 8% | 6% | 77% |
| A510C^{b} | 80% | 71% | 8% | 9% | 7% | 84% |
| | | | | | | |
| **1366^{a}** | 97% | 12% | 39% | 7% | 43% | 91% |
| A510E^{b} | 99% | 9% | 39% | 16% | 36% | 89% |
| A510V^{b} | 78% | 17% | 28% | 16% | 39% | 80% |
| A510C^{b} | 97% | 1% | 39% | 12% | 48% | 96% |
| | | | | | | |
| **6907^{a}** | 85% | 7% | 42% | 17% | 34% | 91% |
| A510E^{b} | 89% | 14% | 35% | 25% | 26% | 94% |
| A510V^{b} | poor conversion | | | | | |
| A510C^{b} | poor conversion | | | | | |

| | | | | | | |
|---|---|---|---|---|---|---|
| ^{a} GTF 2919 (SEQ ID NO:28), 0427 (SEQ ID NO:26), 5926 (SEQ ID NO:14), 0847 (SEQ ID NO:2), 0544 (SEQ ID NO:12), 2379 (SEQ ID NO:6), 5618 (SEQ ID NO:18), 4297 (SEQ ID NO:16), 1366 (SEQ ID NO:24), or 6907 (SEQ ID NO:36). ^{b} A510E/V/C, version of listed GTF (footnote [a]) comprising a substitution with Glu, Val, or Cys at the position corresponding to position 510 of SEQ ID NO:62. ^{c} "Oligomer", gluco-oligosaccharides. | | | | | | |

Based on the data in Table 5, it is apparent that some substitutions in various GTFs at the position corresponding to position 510 of SEQ ID NO:62 can increase a GTF's yield of alpha glucan and/or decrease its leucrose yield in glucan synthesis reactions, for example.

### EXAMPLE 3

### Analysis of the Effects of Two or More Amino Acid Substitutions on Glucosyltransferase Selectivity toward Alpha-Glucan Synthesis

This Example describes the effects of introducing multiple amino acid substitutions to a glucosyltransferase and determining their effect on enzyme selectivity toward alpha-glucan synthesis.

Briefly, certain amino acid substitutions were made to SEQ ID NO:4 (GTF 6855, see Table 1 and Example 1 for description of this glucosyltransferase). These substitutions are listed in Table 6 below. Each variant enzyme was entered into a glucan synthesis reaction with parameters that were the same as, or similar to, the following: vessel, 250-mL indented shake flask agitated at 120 rpm; initial pH, 5.7; reaction volume, 50 mL; sucrose, 75 g/L; GTF, 1.5 mL lysate of *E. coli* cells heterologously expressing enzyme; KH₂PO4, 20 mM; temperature, 30 °C; time, about 20-24 hours. The alpha-1,3 glucan yield of each reaction (as measured via methodology similar to that disclosed in Example 1) is provided in Table 6.

**Table 6. Alpha-1,3 Glucan Yields of GTF 6855 (SEQ ID NO:4) Variants with Multiple Amino Acid Substitutions**

| GTF^{a} | Alpha-1,3 Glucan^{b} Yield^{c} |
|---|---|
| A510D/F607Y/R741S | 72.6% |
| A510D/F607Y/N743S | 79.2% |
| A510D/F607Y/D948G | 88.2% |
| A510D/R741S/D948G | 74.5% |
| A510D/F607Y/R741S/D948G | 82.8% |
| A510E/F607Y/R741S/R1172C | 78.2% |
| A510D/F607Y/D820G/D948G | 87.8% |
| A510D/F607Y/D948G/R1172C | 88.6% |
| A510D/F607Y/N743S/D948G/R1172C | 89.4% |
| A510D/F607Y/R741S/L784Q/F929L/R1172C | 79.3% |

| | |
|---|---|
| ^{a} Each listed GTF is a version of GTF 6855 (SEQ ID NO:4) comprising substitutions at respective positions, where each position number is in correspondence with the residue numbering of SEQ ID NO:62. ^{b} Insoluble alpha-1,3 glucan product. ^{c} Alpha-1,3-glucan yield based on glucosyl. | |

Based on the data in Table 6, it is apparent that introduction of multiple amino acid substitutions to GTF 6855 (SEQ ID NO:4) can increase this enzyme's yield of alpha-1,3-glucan; for example, compare these yields to those of GTF 6855 (SEQ ID NO:4) without substitutions shown in Table 3. Each of the variant GTF enzymes listed in Table 6 also exhibited significant reductions in yields of leucrose, glucose and glucooligomers (data not shown).

It is apparent, for example, that a GTF with multiple substitutions such as at positions corresponding to positions 510 and/or 607 of SEQ ID NO:62 can increase a GTF's yield of alpha glucan.

### EXAMPLE 4

### Analysis of the Effects of Additional Amino Acid Substitution Combinations on Glucosyltransferase Selectivity toward Alpha-Glucan Synthesis

This Example describes the effects of introducing multiple amino acid substitutions to a glucosyltransferase and determining their effect on enzyme selectivity toward alpha-glucan synthesis. While this analysis supplements the analysis disclosed above in Example 3, it is interesting to note that several of the additional amino acid substitution combinations provide modified glucosyltransferases with even higher alpha-1,3-glucan yields.

Briefly, certain combinations of amino acid substitutions were made to SEQ ID NO:4 (GTF 6855, see Table 1 and Example 1 for description of this glucosyltransferase) by site-directed mutagenesis of appropriate DNA templates contained in a plasmid. The plasmid sequences encoding each modified glucosyltransferase were individually sequenced to confirm the intended codon changes. Each combination of substitutions is listed in Table 7 below.

Expression plasmids encoding the modified glucosyltransferases were individually used to transform a *B. subtilis* strain containing nine protease deletions (*amyE*::*xylRPxylAcomK-ermC, degUHy32, oppA,* Δ*spollE3501,* Δ*aprE,* Δ*nprE,* Δ*epr,* Δ*ispA,* Δ*bpr,* Δ*vpr,* Δ*wprA,* Δ*mpr-ybfJ,* Δ*nprB*). Transformed cells were spread onto LB plates supplemented with 5 µg/mL chloramphenicol. Colonies growing on these plates were streaked several times onto LB plates with 25 µg/mL chloramphenicol. Each resulting *Bacillus* strain for expressing a particular variant glucosyltransferase was then grown for 6-8 hours in LB medium containing 25 µg/mL chloramphenicol, and then subcultured into Grants II medium at 30 °C for 2-3 days. The cultures were spun at 15000 g for 30 minutes at 4 °C, and the supernatants were filtered through 0.22-µm filters. The filtered supernatants, each of which contained an expressed secreted variant glucosyltransferase, were aliquoted and frozen at -80 °C, and later used (below) for analyzing alpha-1,3-glucan synthesis activity.

The same amount of each variant enzyme, activity-wise, was entered into a glucan synthesis reaction with parameters that were the same as, or similar to, the following: vessel, 500-mL jacketed reactor with Teflon^{®}-pitched blade turbine (45-degree angle) on a glass stir rod and agitated at 50-200 rpm; initial pH, 5.5; reaction volume, 500 mL; sucrose, 108 g/L; KH₂PO₄, 1 mM; temperature, 39 °C; time, about 18-24 hours; filtrate from a previous alpha-1,3-glucan synthesis reaction, 50 vol%. The alpha-1,3 glucan yield of each reaction (as measured via methodology similar to that disclosed in Example 1) is provided in Table 7.

**Table 7. Alpha-1,3 Glucan Yields of GTF 6855 (SEQ ID NO:4) Variants with Multiple Amino Acid Substitutions**

| GTF^{a} | | | | | | | | | | | | | Alpha-1,3-Glucan^{b} |
|---|---|---|---|---|---|---|---|---|---|---|---|---|---|
| | | | | | | | | | | | | | Yield^{c} |
| A510D | Q588L | F607Y | R741S | D948G | | | R722H | T877K | | M1253I | K1277N | | 88% |
| A510D | Q588L | F607Y | R741S | D948G | | | R722H | T877K | V1188E | M1253I | Q957P | | 92% |
| A510D | Q588L | F607Y | R741S | D948G | | | | T877K | V1188E | M1253I | Q957P | | 91% |
| A510D | Q588L | F607Y | R741S | D948G | | | | | | M1253I | | | 89% |
| A510D | Q588L | F607W | R741S | D948G | | | | | | | | | 91% |
| | Q588L | F607Y | R741S | D948G | | | | | | | | | 91% |
| A510D | Q588L | F607Y | R741S | D948G | N628D | T635A | | T877K | | M1253I | F929L | R1172C | 92% |
| A510D | Q588L | F607W | R741S | D948G | S631T | S710G | R722H | T877K | V1188E | M1253I | | | 94% |
| A510D | Q588L | F607W | R741S | D948G | S631T | S710G | R722H | T877K | V1188E | | | | 93% |
| A510D | Q588L | F607W | R741S | D948G | S631T | S710G | | T877K | V1188E | M1253I | | | 96% |
| A510D | Q588L | F607Y | R741S | D948G | | | | | | | | | 89% |
| A510D | Q588L | F607Y | R741S | D948G | | | | | V1188E | | | | 88% |
| A510D | Q588L | F607W | R741S | D948G | S631T | S710G | | | V1188E | | | | 96% |
| A510D | Q588L | F607W | R741S | D948G | | S710G | R722H | T877K | | M1253I | | | 96% |
| A510D | Q588L | F607Y | R741S | D948G | S631T | | R722H | T877K | V1188E | M1253I | | | 96% |
| A510D | Q588L | F607W | R741S | D948G | S631T | | | T877K | V1188E | M1253I | | | 94% |
| A510D | Q588L | F607W | R741S | D948G | S631T | | | | V1188E | | | | 98% |
| A510D | Q588L | F607Y | R741S | D948G | S631T | | R722H | T877K | V1188E | M1253I | | | 95% |
| A510D | Q588L | F607W | R741S | D948G | | | | | V1188E | M1253I | | | 93% |

| | | | | | | | | | | | | | |
|---|---|---|---|---|---|---|---|---|---|---|---|---|---|
| ^{a} Each listed GTF is a version of GTF 6855 (SEQ ID NO:4) comprising substitutions at respective positions, where each position number is in correspondence with the residue numbering of SEQ ID NO:62. ^{b} Insoluble alpha-1,3 glucan product. ^{c} Alpha-1,3-glucan yield based on glucosyl. | | | | | | | | | | | | | |

Based on the data in Table 7, it is further apparent that introduction of multiple amino acid substitutions to GTF 6855 (SEQ ID NO:4) can increase this enzyme's yield of alpha-1,3-glucan; for example, compare these yields to those of GTF 6855 (SEQ ID NO:4) without substitutions shown in Table 3. Each of the variant GTF enzymes listed in Table 7 also exhibited significant reductions in yields of leucrose, glucose and glucooligomers (data not shown).

It is apparent, for example, that a GTF with multiple substitutions, including those at positions corresponding to positions 588, 607 and 741 of SEQ ID NO:62, can increase a GTF's yield of alpha glucan.

## Claims

1. A non-native glucosyltransferase comprising at least two amino acid substitutions at positions corresponding with amino acid residues Gln-588, Phe-607, or Arg-741 of SEQ ID NO:62,
wherein the non-native glucosyltransferase synthesizes alpha-glucan comprising 1,3-linkages, and
wherein the non-native glucosyltransferase has:
(i) an alpha-glucan yield that is higher than the alpha-glucan yield of a second glucosyltransferase that only differs from the non-native glucosyltransferase at the substitution positions, and/or
(ii) a leucrose yield that is lower than the leucrose yield of the second glucosyltransferase;
wherein the glucosyltransferase comprises a catalytic domain that is at least 90% identical to residues 55-960 of SEQ ID NO:4; and
wherein:
(a) the amino acid substitution at the position corresponding with amino acid residue Gln-588 is with a Leu, Ala, or Val residue;
(b) the amino acid substitution at the position corresponding with amino acid residue Phe-607 is with a Trp or Asn residue; and/or
(c) the amino acid substitution at the position corresponding with amino acid residue Arg-741 is with a Ser or Thr residue.

2. The non-native glucosyltransferase of claim 1, wherein the glucosyltransferase comprises amino acid substitutions at positions corresponding with amino acid residues (A) Gln 588 and Phe 607 of SEQ ID NO:62; or (B) Phe 607 and Arg 741 of SEQ ID NO:62.

3. The non-native glucosyltransferase of claim 1, wherein the glucosyltransferase comprises amino acid substitutions at positions corresponding with amino acid residues Gln-588, Phe-607 and Arg-741 of SEQ ID NO:62.

4. The non-native glucosyltransferase of claim 3, wherein the amino acid substitutions at positions corresponding with amino acid residues Gln-588, Phe-607 and Arg-741 of SEQ ID NO:62 are with Leu, Trp and Ser residues, respectively.

5. The non-native glucosyltransferase of any one of claims 1 to 4, wherein the glucosyltransferase further comprises at least one amino acid substitution at a position corresponding with amino acid residue Ala-510 and/or Asp-948 of SEQ ID NO:62;
wherein:
(i) the amino acid substitution at the position corresponding with amino acid residue Ala-510 is with an Asp, Glu, Ile, or Val residue; and/or
(ii) the amino acid substitution at the position corresponding with amino acid residue Asp-948 is with a Gly, Val, or Ala residue.

6. The non-native glucosyltransferase of any one of claims 1 to 5, wherein the glucosyltransferase further comprises at least one amino acid substitution at a position corresponding with amino acid residue Ser-631, Ser-710, Arg-722, and/or Thr-877 of SEQ ID NO:62;
wherein:
(i) the amino acid substitution at the position corresponding with amino acid residue Ser-631 is with a Thr, Asp, Glu, or Arg residue;
(ii) the amino acid substitution at the position corresponding with amino acid residue Ser-710 is with a Gly, Ala, or Val residue;
(iii) the amino acid substitution at the position corresponding with amino acid residue Arg-722 is with a His or Lys residue; and/or
(iv) the amino acid substitution at the position corresponding with amino acid residue Thr-877 is with a Lys, His, or Arg residue.

7. The non-native glucosyltransferase of any one of claims 1 to 6, wherein the glucosyltransferase further comprises at least one amino acid substitution at a position corresponding with amino acid residue Val-1188, Met-1253, and/or Gln-957 of SEQ ID NO:62;
wherein:
(i) the amino acid substitution at the position corresponding with amino acid residue Val-1188 is with a Glu or Asp residue;
(ii) the amino acid substitution at the position corresponding with amino acid residue Met-1253 is with an Ile, Leu, Ala, or Val residue; and/or
(iii) the amino acid substitution at the position corresponding with amino acid residue Gln-957 is with a Pro residue.

8. The non-native glucosyltransferase of any one of claims 1 to 7, wherein the alpha-glucan is insoluble and comprises at least about 50% alpha-1,3 linkages, and optionally wherein the alpha-glucan has a weight average degree of polymerization (DP_{w}) of at least 100.

9. The non-native glucosyltransferase of claim 8, comprising a catalytic domain that is at least about 95% identical to residues 55-960 of SEQ ID NO:4.

10. The non-native glucosyltransferase of claim 8 or 9, comprising an amino acid sequence that is at least about 90% identical to SEQ ID NO:4.

11. The non-native glucosyltransferase of any one of claims 1 to 10, wherein the non-native glucosyltransferase synthesizes insoluble alpha-1,3-glucan having at least about 90% alpha-1,3-linkages.

12. A polynucleotide comprising a nucleotide sequence encoding a non-native glucosyltransferase according to any one of claims 1 to 11, wherein one or more regulatory sequences are operably linked to the nucleotide sequence.

13. The polynucleotide of claim 12, wherein said one or more regulatory sequences include a promoter sequence.

14. A reaction composition comprising water, sucrose, and a non-native glucosyltransferase according to any one of claims 1 to 11.

15. A method of producing alpha-glucan comprising:
(a) contacting at least water, sucrose, and a non-native glucosyltransferase according to any one of claims 1 to 11, whereby alpha-glucan is produced; and
(b) optionally, isolating the alpha-glucan produced in step (a).

## Patentansprüche

1. Nicht-native Glucosyltransferase, umfassend mindestens zwei Aminosäuresubstitutionen an Positionen, die den Aminosäureresten Gln-588, Phe-607 oder Arg-741 von SEQ ID NO: 62 entsprechen,
wobei die nicht-native Glucosyltransferase alpha-Glucan synthetisiert, das 1,3-Verknüpfungen umfasst, und
wobei die nicht-native Glucosyltransferase aufweist:
(i) eine alpha-Glucan-Ausbeute, die höher ist als die alpha-Glucan-Ausbeute einer zweiten Glucosyltransferase, die sich nur an den Substitutionspositionen von der nicht-nativen Glucosyltransferase unterscheidet, und/oder
(ii) eine Leucroseausbeute, die niedriger ist als die Leucroseausbeute der zweiten Glucosyltransferase;
wobei die Glucosyltransferase eine katalytische Domäne umfasst, die wenigstens 90 % identisch mit den Resten 55-960 von SEQ ID NO: 4 ist; und
wobei:
(a) die Aminosäuresubstitution an der Position, die dem Aminosäurerest Gln-588 entspricht, mit einem Leu-, Ala- oder Val-Rest ist;
(b) die Aminosäuresubstitution an der Position, die dem Aminosäurerest Phe-607 entspricht, mit einem Trp- oder Asn-Rest ist; und/oder
(c) die Aminosäuresubstitution an der Position, die dem Aminosäurerest Arg-741 entspricht, mit einem Ser- oder Thr-Rest ist;

2. Nicht-native Glucosyltransferase nach Anspruch 1, wobei die Glucosyltransferase Aminosäuresubstitutionen an Positionen umfasst, die den Aminosäureresten (A) Gln 588 und Phe 607 von SEQ ID NO: 62; oder (B) Phe 607 und Arg 741 von SEQ ID NO: 62 entsprechen.

3. Nicht-native Glucosyltransferase nach Anspruch 1, wobei die Glucosyltransferase Aminosäuresubstitutionen an Positionen umfasst, die den Aminosäureresten Gln-588, Phe-607 und Arg-741 von SEQ ID NO: 62 entsprechen.

4. Nicht-native Glucosyltransferase nach Anspruch 3, wobei die Aminosäuresubstitutionen an Positionen, die den Aminosäureresten Gln-588, Phe-607 und Arg-741 von SEQ ID NO: 62 entsprechen, Leu-, Trp- bzw. Ser-Reste aufweisen.

5. Nicht-native Glucosyltransferase nach einem der Ansprüche 1 bis 4, wobei die Glucosyltransferase ferner mindestens eine Aminosäuresubstitution an einer Position umfasst, die dem Aminosäurerest Ala-510 und/oder Asp-948 von SEQ ID NO: 62 entspricht;
wobei:
(i) die Aminosäuresubstitution an der Position, die dem Aminosäurerest Ala-510 entspricht, mit einem Asp-, Glu-, Ile- oder Val-Rest ist; und/oder
(ii) die Aminosäuresubstitution an der Position, die dem Aminosäurerest Asp-948 entspricht, mit einem Gly-, Val- oder Ala-Rest ist.

6. Nicht-native Glucosyltransferase nach einem der Ansprüche 1 bis 5, wobei die Glucosyltransferase ferner mindestens eine Aminosäuresubstitution an einer Position umfasst, die dem Aminosäurerest Ser-631, Ser-710, Arg-722 und/oder Thr-877 von SEQ ID NO: 62 entspricht; wobei:
(i) die Aminosäuresubstitution an der Position, die dem Aminosäurerest Ser-631 entspricht, mit einem Thr-, Asp-, Glu- oder Arg-Rest ist;
(ii) die Aminosäuresubstitution an der Position, die dem Aminosäurerest Ser-710 entspricht, mit einem Gly-, Ala- oder Val-Rest ist;
(iii) die Aminosäuresubstitution an der Position, die dem Aminosäurerest Arg-722 entspricht, mit einem His- oder Lys-Rest ist; und/oder
(iv) die Aminosäuresubstitution an der Position, die dem Aminosäurerest Thr-877 entspricht, mit einem Lys-, His- oder Arg-Rest ist.

7. Nicht-native Glucosyltransferase nach einem der Ansprüche 1 bis 6, wobei die Glucosyltransferase ferner mindestens eine Aminosäuresubstitution an einer Position umfasst, die dem Aminosäurerest Val-1188, Met-1253 und/oder Gln-957 von SEQ ID NO: 62 entspricht;
wobei:
(i) die Aminosäuresubstitution an der Position, die dem Aminosäurerest Val-1188 entspricht, mit einem Glu- oder Asp-Rest ist;
(ii) die Aminosäuresubstitution an der Position, die dem Aminosäurerest Met-1253 entspricht, mit einem Ile-, Leu- , Ala- oder Val-Rest ist; und/oder
(iii) die Aminosäuresubstitution an der Position, die dem Aminosäurerest Gln-957 entspricht, mit einem Pro-Rest ist.

8. Nicht-native Glucosyltransferase nach einem der Ansprüche 1 bis 7, wobei das alpha-Glucan unlöslich ist und zu mindestens etwa 50 % alpha-1,3-Bindungen umfasst und wobei das alpha-Glucan gegebenenfalls einen gewichtsmittleren Polymerisationsgrad (DP_{W}) von mindestens 100 aufweist.

9. Nicht native Glucosyltransferase nach Anspruch 8, umfassend eine katalytische Domäne, die zu wenigstens etwa 95 % identisch ist mit den Resten 55-960 von SEQ ID NO: 4.

10. Nicht native Glucosyltransferase nach Anspruch 8 oder 9, umfassend eine Aminosäuresequenz, die zu wenigstens 90 % identisch ist mit SEQ ID NO: 4.

11. Nicht-native Glucosyltransferase nach einem der Ansprüche 1 bis 10, wobei die nicht-native Glucosyltransferase unlösliches alpha-1,3-Glucan mit mindestens etwa 90 % alpha-1,3-Bindungen synthetisiert.

12. Polynukleotid, umfassend eine Nukleotidsequenz, die für eine nicht-native Glucosyltransferase nach einem der Ansprüche 1 bis 11 kodiert, wobei eine oder mehrere regulatorische Sequenzen mit der Nukleotidsequenz wirkverknüpft sind.

13. Polynukleotid nach Anspruch 12, wobei die eine oder die mehreren regulatorischen Sequenzen eine Promotorsequenz beinhalten.

14. Reaktionszusammensetzung, umfassend Wasser, Saccharose und eine nicht-native Glucosyltransferase nach einem der Ansprüche 1 bis 11.

15. Verfahren zur Herstellung von alpha-Glucan, umfassend:
(a) Inkontaktbringen von wenigstens Wasser, Saccharose und einer nicht nativen Glucosyltransferase nach einem der Ansprüche 1 bis 11, wodurch alpha-Glucan hergestellt wird; und
(b) gegebenenfalls Isolieren des bei Schritt (a) hergestellten alpha-Glucans.

## Revendications

1. Glucosyltransférase non native comprenant au moins deux substitutions d'acides aminés à des positions correspondant aux résidus d'acides aminés Gln-588, Phe-607, ou Arg-741 de SEQ ID N° 62,
la glucosyltransférase non native synthétisant de l'alpha-glucane comprenant des liaisons 1,3, et
la glucosyltransférase non native ayant :
(i) un rendement en alpha-glucane supérieur au rendement en alpha-glucane d'une deuxième glucosyltransférase qui ne diffère que de la glucosyltransférase non native aux positions de substitution, et/ou
(ii) un rendement en leucrose inférieur au rendement en leucrose de la deuxième glucosyltransférase ;
la glucosyltransférase comprenant un domaine catalytique qui est au moins à 90 % identique aux résidus 55-960 de SEQ ID N° : 4 ; et
dans laquelle :
(a) la substitution d'acide aminé au niveau de la position correspondant au résidu d'acide aminé Gln-588 est une substitution par un résidu Leu, Ala ou Val ;
(b) la substitution d'acide aminé au niveau de la position correspondant au résidu d'acide aminé Phe-607 est une substitution par un résidu Trp ou Asn ; et/ou
(c) la substitution d'acide aminé au niveau de la position correspondant au résidu d'acide aminé Arg-741 est une substitution par un résidu Ser ou Thr.

2. Glucosyltransférase non native selon la revendication 1, la glucosyltransférase comprenant des substitutions d'acides aminés à des positions correspondant aux résidus d'acides aminés (A) Gln 588 et Phe 607 de SEQ ID NO :62 ; ou (B) Phe 607 et Arg 741 de SEQ ID NO :62.

3. Glucosyltransférase non native selon la revendication 1, la glucosyltransférase comprenant des substitutions d'acides aminés à des positions correspondant aux résidus d'acides aminés Gln-588, Phe-607 et Arg-741 de SEQ ID NO :62.

4. Glucosyltransférase non native selon la revendication 3, les substitutions d'acides aminés aux positions correspondant aux résidus d'acides aminés Gln-588, Phe-607 et Arg-741 de SEQ ID NO :62 étant des substitutions par les résidus Leu, Trp et Ser, respectivement.

5. Glucosyltransférase non native selon l'une quelconque des revendications 1 à 4, la glucosyltransférase comprenant en outre au moins une substitution d'acide aminé à une position correspondant au résidu d'acide aminé Ala-510 et/ou Asp-948 de SEQ ID NO :62 ;
dans laquelle :
(i) la substitution d'acide aminé au niveau de la position correspondant au résidu d'acide aminé Ala-510 est une substitution par un résidu Asp, Glu, Ile ou Val ; et/ou
(ii) la substitution d'acide aminé au niveau de la position correspondant au résidu d'acide aminé Asp-948 est une substitution par un résidu Gly, Val ou Ala.

6. Glucosyltransférase non native selon l'une quelconque des revendications 1 à 5, la glucosyltransférase comprenant en outre au moins une substitution d'acide aminé à une position correspondant au résidu d'acide aminé Ser-631, Ser-710, Arg-722, et/ou Thr-877 de SEQ ID N° :62 ;
dans laquelle :
(i) la substitution d'acide aminé au niveau de la position correspondant au résidu d'acide aminé Ser-631 est une substitution par un résidu Thr, Asp, Glu ou Arg ;
(ii) la substitution d'acide aminé au niveau de la position correspondant au résidu d'acide aminé Ser-710 est une substitution par un résidu Gly, Ala ou Val ;
(iii) la substitution d'acide aminé au niveau de la position correspondant au résidu d'acide aminé Arg-722 est une substitution par un résidu His ou Lys ; et/ou
(iv) la substitution d'acide aminé au niveau de la position correspondant au résidu d'acide aminé Thr-877 est une substitution par un résidu Lys, His ou Arg.

7. Glucosyltransférase non native selon l'une quelconque des revendications 1 à 6, la glucosyltransférase comprenant en outre au moins une substitution d'acide aminé à une position correspondant au résidu d'acide aminé Val-1188, Met-1253 et/ou Gln-957 de SEQ ID N° :62 ;
dans laquelle :
(i) la substitution d'acide aminé au niveau de la position correspondant au résidu d'acide aminé Val-1188 est une substitution par un résidu Glu ou Asp ;
(ii) la substitution d'acide aminé au niveau de la position correspondant au résidu d'acide aminé Met-1253 est une substitution par un résidu Ile, Leu, Ala ou Val ; et/ou
(iii) la substitution d'acide aminé au niveau de la position correspondant au résidu d'acide aminé Gln-957 est une substitution par un résidu Pro.

8. Glucosyltransférase non native selon l'une quelconque des revendications 1 à 7, l'alpha-glucane étant insoluble et comprenant au moins environ 50 % de liaisons alpha-1,3, et facultativement l'alpha-glucane ayant un degré moyen en poids de polymérisation (DP_{W}) d'au moins 100.

9. Glucosyltransférase non native selon la revendication 8, comprenant un domaine catalytique qui est au moins à 95 % environ identique aux résidus 55-960 de SEQ ID N° : 4.

10. Glucosyltransférase non native selon la revendication 8 ou 9, comprenant une séquence d'acides aminés qui est au moins à 90 % environ identique à SEQ ID N° : 4.

11. Glucosyltransférase non native selon l'une quelconque des revendications 1 à 10, la glucosyltransférase non native synthétisant de l'alpha-1,3-glucane insoluble ayant au moins environ 90 % de liaisons alpha-1,3.

12. Polynucléotide comprenant une séquence nucléotidique codant pour une glucosyltransférase non native selon l'une quelconque des revendications 1 à 11, une ou plusieurs séquences régulatrices étant liées de manière fonctionnelle à la séquence nucléotidique.

13. Polynucléotide selon la revendication 12, lesdites une ou plusieurs séquences régulatrices comprenant une séquence promotrice.

14. Composition réactionnelle comprenant de l'eau, du saccharose et une glucosyltransférase non native selon l'une quelconque des revendications 1 à 11.

15. Procédé de production d'alpha-glucane comprenant :
(a) la mise en contact au moins d'eau, de saccharose et d'une glucosyltransférase non native selon l'une quelconque des revendications 1 à 11, moyennant quoi un alpha-glucane étant produit ; et
(b) éventuellement, l'isolement de l'alpha-glucane produit à l'étape (a).
